(19) Europäisches Patentamt / European Patent Office / Office européen des brevets

(11) **EP 4 003 404 B1**

(12) **FASCICULE DE BREVET EUROPEEN**

(45) Date de publication et mention de la délivrance du brevet:
**14.01.2026   Bulletin 2026/03**

(21) Numéro de dépôt: **20746943.8**

(22) Date de dépôt: **24.07.2020**

(51) Classification Internationale des Brevets (IPC):
**A61K 38/22** *(2006.01)*    **A61L 2/16** *(2006.01)*
**A61P 31/04** *(2006.01)*    **A61K 38/17** *(2006.01)*

(52) Classification Coopérative des Brevets (CPC):
**A61K 38/22; A61K 38/1703; A61K 38/2242; A61P 31/04**

(86) Numéro de dépôt international:
**PCT/EP2020/071029**

(87) Numéro de publication internationale:
**WO 2021/018792 (04.02.2021 Gazette 2021/05)**

(54) **L'OSTEOCRINE, LA LEBETINE OU L'ANP POUR DETRUIRE LES BIOFILMS BACTERIENS**

OSTEOCRIN, LEBETIN ODER ANP ZUR ZERSTÖRUNG BAKTERIELLER BIOFILME

OSTEOCRIN, LEBETIN OR ANP FOR DESTROYING BACTERIAL BIOFILMS

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **26.07.2019   FR 1908511**

(43) Date de publication de la demande:
**01.06.2022   Bulletin 2022/22**

(73) Titulaire: **Université de Rouen Normandie**
**76130 Mont-Saint-Aignan (FR)**

(72) Inventeurs:
• **LESOUHAITIER, Olivier**
**27340 PONT DE L'ARCHE (FR)**
• **FEUILLOLEY, Marc**
**27930 LE BOULAY MORIN (FR)**
• **CLAMENS, Thomas**
**Londres SW7 5RR (GB)**
• **RODRIGUES, Sophie**
**27000 EVREUX (FR)**
• **LAURENCY, Sylvie**
**27000 EVREUX (FR)**
• **LOUIS, Mélissande**
**27630 FOURGES VEXIN-SUR-EPTE (FR)**

(74) Mandataire: **Ipsilon**
**12 Avenue d'Italie**
**75013 Paris (FR)**

(56) Documents cités:
**WO-A1-2009/033701     WO-A1-88/06596**
**US-B2- 7 033 997**

• **TOURKI BOCHRA ET AL: "Lebetin 2, a Snake Venom-Derived Natriuretic Peptide, Attenuates Acute Myocardial Ischemic Injury through the Modulation of Mitochondrial Permeability Transition Pore at the Time of Reperfusion",** PLOS ONE, vol. 11, no. 9, 12 September 2016 (2016-09-12), pages e0162632, XP093100822, Retrieved from the Internet <URL:https://journals.plos.org/plosone/article/file?id=10.1371/journal.pone.0162632&type=printable> DOI: 10.1371/journal.pone.0162632
• **THIBAUT ROSAY ET AL: "ABSTRACT", MBIO,** vol. 6, no. 4, 1 September 2015 (2015-09-01), XP055674773, DOI: 10.1128/mBio.01033-15
• **GANNESEN A V ET AL: "Regulation of Formation of Monospecies and Binary Biofilms by Human Skin Microbiota Components,Staphylococcus epidermidisandStaphylococcus aureus, by Human Natriuretic Peptides", MICROBIOLOGY,** CONSULTANTS BUREAU, NEW YORK, NY, US, vol. 87, no. 5, 3 October 2018 (2018-10-03), pages 597 - 609, XP036607212, ISSN: 0026-2617, [retrieved on 20181003], DOI: 10.1134/S0026261718050090

- **ANDREI VLADISLAVOVICH GANNESEN ET AL: "Regulation of Monospecies and Mixed Biofilms Formation of Skin Staphylococcus aureus and Cutibacterium acnes by Human Natriuretic Peptides", FRONTIERS IN MICROBIOLOGY, vol. 9, 10 December 2018 (2018-12-10), XP055674888, DOI: 10.3389/fmicb.2018.02912**
- **OLIVIER LESOUHAITIER ET AL: "Host Peptidic Hormones Affecting Bacterial Biofilm Formation and Virulence", JOURNAL OF INNATE IMMUNITY, vol. 11, no. 3, 5 November 2018 (2018-11-05), CH, pages 227 - 241, XP055730751, ISSN: 1662-811X, DOI: 10.1159/000493926**

Remarques:
Le document complet y compris le(s) table(s) de référence et le(s) listage(s) de séquence sont téléchargeables sur le site internet de l'OEB

**Description**

La présente invention concerne la destruction de biofilms bactériens, en particulier dans le cadre d'infections chroniques bactériennes.

La résistance des bactéries aux antibiothérapies est largement attribuée à leur capacité à passer d'une forme de vie planctonique à une forme de vie communautaire, nommée biofilm, dans les tissus de l'hôte infecté. Le biofilm permet aux bactéries de se protéger contre les molécules antimicrobiennes et les défenses immunitaires de l'hôte, permettant ainsi aux pathogènes de survivre dans des environnements hostiles en leur conférant une résistance à diverses molécules antibactériennes. De plus, un faible pourcentage de cellules bactériennes qualifiées de « cellules persistantes » se maintiennent au sein d'un biofilm exposé aux antibiotiques devenant donc temporairement résistantes et responsables de la reprise d'infections récurrentes.

Le pathogène opportuniste *Pseudomonas aeruginosa* est une cause majeure de mortalité chez les patients immunodéprimés, en particulier chez les patients souffrant de mucoviscidose.

Le traitement des infections sévères à *P. aeruginosa* est réalisé au moyen de plusieurs antibiotiques présentant, pour les plus efficaces (la tobramycine, la colistine, la ciprofloxacine, la gentamicine, la nétilmicine et l'amikacine) un index thérapeutique étroit dû à l'existence d'une néphrotoxicité et d'une ototoxicité. Par ailleurs, ces traitements engendrent dans la durée l'apparition de souches résistantes aux antibiotiques.

C'est la raison pour laquelle *P. aeruginosa* a été classé dans le groupe de bactéries ESKAPE (Santajit et Indrawattana (2016) Biomed. Res. Int. 2016 :2475067), regroupant les pathogènes les plus critiques en termes de résistance aux antibiotiques et nécessitant la découverte de nouvelles molécules ou stratégies thérapeutiques. Plus récemment, l'OMS a classé *P. aeruginosa* dans le top 3 des priorités en terme de bactéries critiques pour leurs antibio-résistances (aux carbapénèmes en particulier).

Lesouhaitier et al. (2019, J. Innate Immun, 11 :227-241) décrivent des hormones peptidiques capables de modifier la formation de biofilm bactériens, ainsi que leurs mécanismes d'action.

La présente invention vise à répondre à ce besoin.

La présente invention résulte de la découverte inattendue, par les inventeurs, que l'hormone « *Peptide Natriurétique Auriculaire* » ou ANP *(« Atrial Natriuretic Peptide »* en anglais) est capable, à faible dose (dès 0.1 nM), et très rapidement (dès 30 min après administration), de détruire environ 80% des biofilms de *P. aeruginosa* pré-établis et ceci de manière dose-dépendante. Les inventeurs ont également démontré que d'autres peptides appartenant à la famille des peptides natriurétiques, plus particulièrement l'ostéocrine, la lébétine 2α et la lébétine 1β, étaient également capables à faible dose de détruire des biofilms de *P. aeruginosa* pré-établis.

Les peptides natriurétiques constituent une famille d'hormones initialement identifiées pour leurs activités cardiovasculaires, osmorégulatrices, de régulation de la pression artérielle et d'élimination de sodium par les reins (natriurèse, d'où leur nom). Les trois membres principaux de cette famille sont l'ANP, le peptide « *Brain Natriuretic Peptide* » ou BNP et le peptide « *C-type Natriuretic Peptide* » ou CNP. La longueur de ces peptides est comprise entre 22 et 38 acides aminés et leur structure est caractérisée, pour la plupart des membres, par un pont disulfure formant une boucle de 17 acides aminés conférant à cette molécule une forme dite « omega ».

Il a été précédemment montré que l'ANP, le BNP et le CNP empêchaient la formation de biofilm de *P. aeruginosa* (Desriac et al. The natriuretic peptide hormones prevent Pseudomonas aeruginosa biofilm formation through a specific bacterial target. 16th international conference on Pseudomonas, Sep 2017, Liverpool, United Kingdom; Rosay et al. (2015) MBio 6 :6 ; Desriac et al. (2018) Pathogens 7 :47).

Toutefois, aucune action de ces peptides sur des biofilms déjà établis n'a été montrée à ce jour.

Les inventeurs ont en outre ici montré que la capacité de l'ANP de disperser les biofilms déjà établis n'était pas associée ou due à une capacité de l'ANP à tuer les bactéries et est sans effet sur la virulence intrinsèque des bactéries. Ce mode d'action particulier permet d'éviter l'émergence de souches bactériennes résistantes à cette molécule.

Par ailleurs, les inventeurs ont montré que lorsque l'ANP, à faible dose, est utilisé en combinaison avec un antibiotique, tel que la tobramycine ou la ciprofloxacine, lui-même utilisé à faible dose, plus de 97% du biofilm établi est détruit.

Ainsi, l'ANP et les antibiotiques présentent une action synergique sur la destruction des biofilms bactériens.

L'invention est telle que définie dans le jeu de revendications.

La présente invention concerne donc un peptide natriurétique choisi dans le groupe constitué de (i) l'ostéocrine, un propeptide d'ostéocrine ou un dérivé de l'ostéocrine, (ii) une lébétine , un fragment de lébétine ou un dérivé de lébétine ou d'un fragment de lébétine, et (iii) un peptide ANP, propeptide d'ANP ou dérivé de peptide ANP, pour son utilisation dans une méthode de traitement thérapeutique d'une infection bactérienne, en particulier d'une infection bactérienne chronique, associée à un biofilm bactérien chez un sujet, dans laquelle ledit peptide natriurétique, en particulier ledit peptide ANP, propeptide d'ANP ou dérivé de peptide ANP, disperse ledit biofilm bactérien, dans laquelle le dérivé d'ostéocrine est une ostéocrine dans laquelle un ou plusieurs résidus d'acide aminé a été modifié chimiquement ou un variant peptidique d'ostéocrine,

dans laquelle le dérivé de lébétine ou d'un fragment de lébétine est une lébétine ou un fragment de lébétine dans laquelle/lequel un ou plusieurs résidus d'acide aminé a été modifié chimiquement ou un variant peptidique de lébétine ou de fragment de lébétine, et dans laquelle ledit dérivé de peptide ANP est une forme tronquée d'ANP correspondant aux acides aminés 4 ou 5 à 28 de la forme entière de l'ANP, une forme ayant une structure qui est un dimère antiparallèle de l'ANP, un polymère constitué de monomères d'ANP, un peptide ANP dans lequel 1 à 4 acides aminés ont été supprimés, substitués ou ajoutés, un peptide ANP dans lequel a été substitué l'acide aminé Phe de la boucle en position 8, un peptide ANP dans lequel a été substitué l'acide aminé Phe en position 26 ou l'acide aminé Ser en position 25, l'urodilatine, le fsANP ou une version linéarisée de l'ANP avec clivage du pont disulfure.

Dans un mode de réalisation particulier, ledit peptide natriurétique, en particulier ledit peptide ANP, propeptide d'ANP ou dérivé de peptide ANP, est utilisé en combinaison avec un antibiotique.

Un autre objet de l'invention concerne une composition pharmaceutique comprenant (A) un peptide natriurétique choisi dans le groupe constitué d'une lébétine, un fragment de lébétine et un dérivé de lébétine ou d'un fragment de lébétine, et (B) un antibiotique, dans laquelle le dérivé de lébétine ou d'un fragment de lébétine est une lébétine ou un fragment de lébétine dans laquelle/lequel un ou plusieurs résidus d'acide aminé a été modifié chimiquement ou un variant peptidique.

La demande décrit également une composition pharmaceutique comprenant (A) un peptide natriurétique choisi dans le groupe constitué de (i) l'ostéocrine, un propeptide d'ostéocrine ou un dérivé de l'ostéocrine, (ii) une lébétine, un fragment de lébétine ou un dérivé de lébétine ou d'un fragment de lébétine, et (iii) un peptide ANP, propeptide d'ANP ou dérivé de peptide ANP, et (B) un antibiotique, pour son utilisation dans une méthode de traitement thérapeutique d'une infection bactérienne, en particulier d'une infection bactérienne chronique, associée à un biofilm bactérien chez un sujet.

Un autre objet de l'invention concerne une combinaison pharmaceutique antibactérienne comprenant (A) un peptide natriurétique choisi dans le groupe constitué de (i) l'ostéocrine, un propeptide d'ostéocrine ou un dérivé de l'ostéocrine, (ii) une lébétine , un fragment de lébétine ou un dérivé de lébétine ou d'un fragment de lébétine, et (iii) un peptide ANP, propeptide d'ANP ou dérivé de peptide ANP, et (B) un antibiotique pour une utilisation simultanée, séparée ou séquentielle dans le traitement thérapeutique d'une infection bactérienne, en particulier d'une infection bactérienne chronique, associée à un biofilm bactérien chez un sujet, dans laquelle ledit peptide natriurétique disperse ledit biofilm bactérien,

dans laquelle le dérivé d'ostéocrine est une ostéocrine dans laquelle un ou plusieurs résidus d'acide aminé a été modifié chimiquement ou un variant peptidique d'ostéocrine,
dans laquelle le dérivé de lébétine ou d'un fragment de lébétine est une lébétine ou un fragment de lébétine dans laquelle/lequel un ou plusieurs résidus d'acide aminé a été modifié chimiquement ou un variant peptidique de lébétine ou de fragment de lébétine, et dans laquelle ledit dérivé de peptide ANP est une forme tronquée d'ANP correspondant aux acides aminés 4 ou 5 à 28 de la forme entière de l'ANP, une forme ayant une structure qui est un dimère antiparallèle de l'ANP, un polymère constitué de monomères d'ANP, un peptide ANP dans lequel 1 à 4 acides aminés ont été supprimés, substitués ou ajoutés, un peptide ANP dans lequel a été substitué l'acide aminé Phe de la boucle en position 8, un peptide ANP dans lequel a été substitué l'acide aminé Phe en position 26 ou l'acide aminé Ser en position 25, l'urodilatine, le fsANP ou une version linéarisée de l'ANP avec clivage du pont disulfure.

La présente invention concerne également l'utilisation *in vitro* ou *ex vivo* d'un peptide natriurétique choisi dans le groupe constitué d (i) l'ostéocrine, un propeptide d'ostéocrine ou un dérivé de l'ostérocrine, (ii) une lébétine , un fragment de lébétine ou un dérivé de lébétine ou d'un fragment de lébétine, et (iii) un peptide ANP, un propeptide d'ANP ou un dérivé de peptide ANP, pour disperser un biofilm bactérien, dans laquelle le dérivé d'ostéocrine est une ostéocrine dans laquelle un ou plusieurs résidus d'acide aminé a été modifié chimiquement ou un variant peptidique d'ostéocrine,
dans laquelle le dérivé de lébétine ou d'un fragment de lébétine est une lébétine ou un fragment de lébétine dans laquelle/lequel un ou plusieurs résidus d'acide aminé a été modifié chimiquement ou un variant peptidique de lébétine ou de fragment de lébétine, et dans laquelle ledit dérivé de peptide ANP est une forme tronquée d'ANP correspondant aux acides aminés 4 ou 5 à 28 de la forme entière de l'ANP, une forme ayant une structure qui est un dimère antiparallèle de l'ANP, un polymère constitué de monomères d'ANP, un peptide ANP dans lequel 1 à 4 acides aminés ont été supprimés, substitués ou ajoutés, un peptide ANP dans lequel a été substitué l'acide aminé Phe de la boucle en position 8, un peptide ANP dans lequel a été substitué l'acide aminé Phe en position 26 ou l'acide aminé Ser en position 25, l'urodilatine, le fsANP ou une version linéarisée de l'ANP avec clivage du pont disulfure.

## Description détaillée de l'invention

*Peptides natriurétiques*

Par "peptide natriurétique", on entend ici un membre de la famille d'hormones et neurohormones eucaryotes composée de 3 principaux membres : le peptide natriurétique auriculaire (« *Atrial Natriuretic Peptide* ») ou ANP, le peptide natriurétique de type B (« *Brain Natriuretic Peptide* ») ou BNP et le peptide natriurétique de type C (« *C-type Natriuretic Peptide* ») ou CNP. Ces peptides sont principalement synthétisés et libérés par les cardiomyocytes et les cellules endothéliales. A côté de ces 3 principaux membres, la famille des peptides natriurétiques incluent également l'ostéocrine ainsi que des peptides « natriurétiques-like » tels que la lébétine.

Ces divers peptides natriurétiques présentent une certaine similarité en terme de séquence. Tous, exceptés l'ostéocrine et la lébétine 1β, possèdent en effet une boucle de 17 acides aminés formée par un pont disulfure. Parmi les 17 acides aminés qui forment cette boucle, 11 sont communs à l'ANP et à la lébétine 2α. L'ostéocrine présente également une signature de séquence conservée dans cette région, avec 7 acides aminées communs avec l'ANP. Cette région conservée de la séquence des peptides natriurétiques permet leur liaison aux récepteurs humains aux peptides natriurétiques (NPR-A, NPR-B et NPR-C). De plus, il a été montré que l'ANP, l'ostéocrine et la lébétine 2α se lient avec une forte affinité sur le senseur AmiC de P. aeruginosa (Rosay et al. (2015) mBio 25 :e01033-15). En revanche, de nombreuses différences dans la séquence en acides aminés de leurs extrémités C et N terminales sont observées, expliquant leurs activités physiologiques différentes. Il est intéressant de noter qu'outre les produits finaux actifs présentés ci-dessous, la synthèse, la maturation et la dégradation partielle des peptides natriurétiques peuvent aboutir à la production de nombreux variants possédant une activité physiologique. Il existe de nombreux membres de cette famille chez l'homme et dans le règne animal : l'urodilatine, décrite ci-dessous, l'uroguanyline, la guanyline, le VNP, le DNP et le KNP.

Le peptide natriurétique ventriculaire (VNP) a initialement été identifié chez l'anguille comme étant un peptide possédant des fonctions physiologiques semblables à celles de l'ANP et qui a, depuis, été identifié chez de nombreuses espèces de poissons (Kawakoshi et al. (2004) J. Mol. Endocrinol. 32 :547-555).

Le dendroapsis natriuretic peptide (DNP) a été identifié dans le venin du serpent mamba vert (Schweitz et al. (1992) J. Biol. Chem. 267 :13928-13932) et le Krait natriuretic peptide (KNP) a été identifié dans le venin du serpent Bungarus flaviceps (Sridharan et al. (2015) Biochem. J. 469 :255-266).

De nombreux autres composés similaires aux peptides natriurétiques sont régulièrement identifiés dans les venins de vipères comme les lébétines isolées du venin de *Macrovipera lebetina,* de grands reptiles comme les dragons de Komodo (Natriuretic peptide toxin Var2) (Fry et al. (2009) Proc. Natl. Acad. Sci. USA 106 :8969-8974) et, de même, dans le venin de scorpions (Alves et al. (2013) Toxicon 74 :19-26). Ils sont généralement appelés peptides à activités natriurétiques-like. Parmi ceux-ci, les peptides natriurétiques préférés sont les lébétines L2α (Gly1-Gly38) et L1β (Asp2-Gly13). Enfin, il est décrit chez la bactérie *Escherichia coli* la présence d'une entérotoxine dénommée ST (« heat-stable enterotoxin ») qui présente une structure peptidique proche de la guanyline et de l'uroguanyline humaines et qui présente une activité natriurétique-like.

Le peptide natriurétique utilisé dans le cadre de l'invention est sélectionné dans le groupe constitué de (i) l'ostéocrine, un propeptide d'ostéocrine ou un dérivé d'ostéocrine, (ii) une lébétine, un fragment de lébétine ou un dérivé de lébétine ou d'un fragment de lébétine, et (iii) un peptide ANP, un propeptide d'ANP ou un dérivé de peptide ANP.

Par "ANP", on entend ici le peptide correspondant à la principale forme mature issue du clivage du fragment N-terminal de la pro-hormone proANP.

Typiquement l'ANP humaine est un peptide de 28 acides aminés issu du clivage du fragment N-terminal de la pro-hormone proANP de 126 acides aminés. L'ANP humaine est formée des acides aminés 99 à 126 de la pro-hormone proANP.

L'ANP est un peptide très conservé dans sa séquence en acides aminés puisque cette séquence est identique entre l'homme, les singes, les gorilles, le cochon, les chevaux et les moutons par exemple (Takei et al. (2011) General and Comparative Endocrinology 171 :258-266). Toutefois, chez de nombreuses espèces animales qui l'expriment, quelques différences de séquence sont observées. Ainsi, le rat, la souris et le lapin ont un acide aminé différent en position 12 (Isoleucine à la place d'une Méthionine chez l'homme). De même, l'éléphant présente un ANP raccourci à 27 aminés et deux acides aminés différents par rapport à l'homme (Takei et al. (2011) General and Comparative Endocrinology 171 :258-266).

Par « propeptide d'ANP », on entend ici tout peptide issu de l'expression du gène codant le preproANP, de la maturation et des clivages, éventuellement successifs, du preproANP, et comprenant la séquence peptidique de l'ANP.

Le terme « propeptide d'ANP » inclut ainsi la pro-hormone proANP (typiquement de 126 acides aminés chez l'homme) portant l'ANP dans sa partie C-terminale, la préprohormone preproANP (typiquement de 151 acides aminés chez l'homme), qui correspond à la protéine produite chez l'homme par le gène NPPA. Le preproANP porte un peptide signal qui est clivé pour former le proANP.

Dans le contexte de la présente invention, le terme « propeptide d'ANP » inclut également tout peptide issu de la maturation et des clivages du preproANP et du proANP.

L'ANP consiste typiquement en la séquence d'acides aminés SLRRSSCFGGRMDRIGAQSGLGCNSFRY (SEQ ID NO: 1), avec un pont disulfure localisé entre les acides aminés 7 et 23.

Le proANP consiste typiquement en la séquence d'acides aminés NPMYNAVSNADLMDFKNLLDHLEEKMP

LEDEVVPPQVLSEPNEEAGAALSPLPEVPPWT GEVSPAQRDGGALGRGPWDSSDRSALLKSKLRALLTAPRSLRRS SCFGGRMDRIGAQS GLGCNSFRY (SEQ ID NO : 3).

Le preproANP consiste typiquement en la séquence d'acides aminés

MSSFSTTTVSFLLLLLAFQLLGQTRANPMYNAVSNADLMDFKNLLDHLEEKMPLEDEVVPP

QVLSEPNEEAGAALSPLPEVPPWTGEVSPAQRDGGALGRGPWDSSDRSALLKSKLRAL

LTAPRSLRRSSCFGGRMDRIGAQSGLGCNSFRY (SEQ ID NO : 4).

Par « dérivé d'ANP », on entend ici des dérivés naturels ou synthétiques de l'ANP.

Par « dérivé d'ANP », on entend ici un peptide ANP tel que défini ci-dessus dans lequel un ou plusieurs résidus d'acide aminé a été modifié chimiquement, par exemple par alkylation, acylation, amidation, méthoxylation, formation d'ester, formation d'amide, insertion d'un substituant lipophile, ajout de polyéthylène glycol (PEG), ajout d'une chaine glucidique.

Par « dérivé d'ANP », on entend ici également des formes tronquées d'ANP correspondant aux acides aminés 4 ou 5 à 28 de la forme entière de l'ANP, des formes ayant une structure qui est un dimère antiparallèle de l'ANP, en particulier l'homodimère β-ANP, un polymère constitué de monomères d'ANP.

Par « dérivé d'ANP », on entend aussi des variants peptidiques d'ANP, c'est-à-dire des formes intermédiaires obtenues au cours de la synthèse, la maturation et la dégradation partielle de l'ANP tel que défini ci-dessus, ainsi que des peptides ANP dans lesquels 1 à 12, en particulier 1 à 10, en particulier 1 à 5, en particulier 1 à 4, en particulier 1 à 3, plus particulièrement 1 ou 2 acides aminés ont été supprimés, substitués ou ajoutés et conservant l'activité de l'ANP sauvage.

Dans un mode de réalisation particulier, le dérivé d'ANP est un peptide ANP dans lequel a été substitué l'acide aminé Phénylalanine (Phe) de la boucle en position 8 (et remplacé par un acide aminé Alanine par exemple).

Dans un autre mode de réalisation particulier, le dérivé d'ANP est un peptide ANP dans lequel a été substitué l'acide aminé Phénylalanine (Phe) en position 26 ou l'acide aminé Sérine en position 25, deux acides aminés responsables de la dégradation de l'ANP sauvage (Ichiki and Burnett (2017) Circ J. 81:913-919).

Dans un mode de réalisation particulier, le dérivé d'ANP est une forme allongée de l'ANP, l'urodilatine, de séquence TAPRSLRRSSCFGGRMDRIGAQSGLGCNSFRY (SEQ ID NO : 2), avec un pont disulfure entre les acides aminés 11 et 27. Ce peptide correspond à un ANP allongé de 4 acides aminés du coté N-terminal.

Dans un autre mode de réalisation particulier, le dérivé d'ANP est une forme allongée du peptide ANP de 12 acides aminés, tel que le fsANP qui est une forme allongée de l'ANP de 12 acides aminés du côté C-terminal et qui est présente chez les personnes portant une mutation dans le gène codant l'ANP (Dickey et al. (2009) J Biol Chem 284:19196-19202).

Dans un autre mode de réalisation particulier, le dérivé d'ANP est une version linéarisée de l'ANP, avec clivage du pont disulfure (initialement localisé entre les acides aminés 7 et 23). Typiquement, les dérivés d'ANP pouvant être utilisés dans le cadre de la présente invention sont de préférence modifiés par rapport à l'ANP de manière à avoir une ½ vie plus longue que l'ANP et/ou d'avoir une affinité plus faible que l'ANP pour les récepteurs endogènes humains de l'ANP.

Par « ostéocrine », on entend ici une hormone agissant comme régulateur de la croissance dendritique dans le cortex cérébral en développement en réponse à une expérience sensorielle.

Typiquement l'ostéocrine humaine est un peptide de 50 acides aminés issu du clivage du fragment N-terminal de la pro-hormone pro-ostéocrine de 106 acides aminés. L'ostéocrine humaine est formée des acides aminés 83 à 132 de la pro-hormone pro-ostéocrine.

Par « propeptide d'ostéocrine », on entend ici la pro-hormone pro-ostéocrine de 106 acides aminés portant l'ostéocrine dans sa partie C-terminale et/ou la préprohormone preproosteocrine de 132 acides aminés, qui correspond à la protéine produite, chez l'homme par le gène OSTN. La prépro-ostéocrine porte un peptide signal qui est clivé pour former la pro-ostéocrine de 106 acides aminés.

L'ostéocrine consiste typiquement en la séquence d'acides aminés SFSGFGSPLDRLSAGSVDHKGKQRKV VDHPKRRFGIPMDRIGRNRLSNSR (SEQ ID NO : 5).

La pro-ostéocrine consiste typiquement en la séquence d'acides aminés VDVTTTEAFDSGVIDVQSTPTVR EEKSATDLTAKLLLLDELVSLENDVIETKKKRSFSGFG SPLDRLSAGSVDHKGKQRKVVDHPKRRFGIPM-DRIGRNRLSNSRG (SEQ ID NO : 6).

La prépro-ostéocrine consiste typiquement en la séquence d'acides aminés MLDWRLASAHFILAVTLTLWSS GKVLSVDVTTTEAFDSGVIDVQSTPTVREEKSATDLTA KLLLLDELVSLENDVIETKKKRSFSGFGSPLDRLSAGSVDH KGKQRKVVDHPKRRFGIPM DRIGRNRLSNSRG (SEQ ID NO : 7).

Dans le contexte de la présente invention, le terme « propeptide d'ostéocrine » inclut également tout peptide issu de la maturation et des clivages de la prépro-ostéocrine et de la pro-ostéocrine.

Par « dérivé d'ostéocrine », on entend ici des dérivés naturels ou synthétiques de l'ostéocrine.

Par « dérivé d'ostéocrine », on entend ici une ostéocrine telle que définie ci-dessus dans laquelle un ou plusieurs résidus d'acide aminé a été modifié chimiquement, par exemple par alkylation, acylation, amidation, méthoxylation, formation d'ester, formation d'amide, insertion d'un substituant lipophile, ajout de polyéthylène glycol (PEG), ajout d'une chaine glucidique.

Par « dérivé d'ostéocrine », on entend aussi des variants peptidiques d'ostéocrine, c'est-à-dire des formes intermédiaires obtenues au cours de la synthèse, la maturation et la dégradation partielle de l'ostéocrine telle que définie ci-dessus.

Par « lébétine », on entend ici un peptide issu du venin de vipère *Macrovipera lebetina.*

La lébétine telle que définie ci-dessus est de préférence la lébétine 2α ou la lébétine 1β.

Par « lébétine 2α », on entend ici un peptide produit par la vipère *Macrovipera lebetina* décrit dans Barbouche et al. (1996) FEBS Lett. 392 :6-10.

Typiquement la lébétine 2α est un peptide de 38 acides aminés.

La lébétine 2α consiste typiquement en la séquence d'acides aminés GDNKPPKKGPPNGCFGHKIDRIGSHSGLGCNKVDDNKG (SEQ ID NO : 8) avec un pont disulfure localisé entre les acides aminés 14 et 30.

Par « lébétine 1β », on entend ici un peptide produit par la vipère *Macrovipera lebetina* décrit dans Barbouche et al. (1998) Toxicon 36(12):1939-47.

Typiquement la lébétine 1β est un peptide de 12 acides aminés.

La lébétine 1β consiste typiquement en la séquence d'acides aminés DNKPPKKGPPNG (SEQ ID NO : 9).

Par « fragment de lébétine », on désigne ici un fragment d'au moins 10 acides aminés de ladite lébétine, de préférence de la lébétine 2α telle que définie ci-dessus ou de la lébétine 1β telle que définie ci-dessus.

Ledit fragment de lébétine comprend de préférence au moins 10 acides aminés, de préférence au moins 12 acides aminés, de préférence au moins 14 acides aminés, de préférence au moins 16 acides aminés et/ou au plus 30 acides aminés, de préférence au plus 25 acides aminés, de préférence au plus 20 acides aminés.

Un fragment de lébétine 2α comprend par exemple les acides aminés 4 à 10 de la séquence SEQ ID NO: 8, de préférence les acides aminés 2 à 13 de la séquence SEQ ID NO: 8.

Un fragment de lébétine 2α consiste par exemple en les acides aminés 1 à 13 de la séquence SEQ ID NO: 8.

La lébétine 1β de séquence SEQ ID NO: 9 est également un fragment de lébétine 2α consistant en les acides aminés 2 à 13 de la séquence SEQ ID NO: 8.

Par « dérivé de lébétine ou d'un fragment de lébétine », on entend ici des dérivés naturels ou synthétiques de la lébétine ou d'un fragment de lébétine.

Par « dérivé de lébétine ou d'un fragment de lébétine », on entend ici une lébétine telle que définie ci-dessus ou un fragment de lébétine tel que défini dans laquelle/lequel un ou plusieurs résidus d'acide aminé a été modifié chimiquement, par exemple par alkylation, acylation, amidation, méthoxylation, formation d'ester, formation d'amide, insertion d'un substituant lipophile, ajout de polyéthylène glycol (PEG), ajout d'une chaine glucidique.

Par « dérivé de lébétine ou d'un fragment de lébétine », on entend aussi des variants peptidiques de lébétine ou d'un fragment de lébétine, c'est-à-dire des formes intermédiaires obtenues au cours de la synthèse, la maturation et la dégradation partielle de la lébétine ou d'un fragment de lébétine telle que définie ci-dessus.

Par « autre peptide natriurétique », on entend ici un peptide natriurétique qui n'est pas l'ANP, un propeptide d'ANP ou un dérivé d'ANP (tel que l'urodilatine).

*Biofilm bactérien et infection bactérienne*

Par « infection bactérienne », on entend ici une infection due à une ou plusieurs espèces de bactéries, en particulier des bactéries à Gram négatif et/ou à Gram positif.

Dans un mode de réalisation particulier, l'infection bactérienne est une infection bactérienne chronique.

Dans un mode de réalisation particulier, l'infection bactérienne est une infection à une bactérie à Gram négatif.

Dans un mode de réalisation particulier, l'infection bactérienne est une infection à une bactérie du genre *Pseudomonas,* plus particulièrement de l'espèce *Pseudomonas aeruginosa.*

Dans un autre mode de réalisation particulier, l'infection bactérienne est une infection à une bactérie à Gram positif.

Dans un mode de réalisation particulier, l'infection bactérienne est une infection à une bactérie du genre *Staphylococcus,* plus particulièrement de l'espèce *Staphylococcus aureus.*

Par « biofilm bactérien », on entend ici une communauté de bactéries adhérant entre elles et à une surface. La production de biofilm est caractérisée par la sécrétion d'une matrice qui est susceptible d'adhérer à de nombreux types de surface, et permettre la cohésion et la protection des bactéries constituant cette structure.

Dans le cadre de l'invention, le biofilm bactérien comprend au moins la ou les espèces bactériennes responsables de l'infection à traiter.

Dans le cadre de l'invention, ledit peptide natriurétique, en particulier ledit peptide ANP, propeptide d'ANP ou dérivé de peptide ANP, disperse le biofilm bactérien.

Par « dispersion du biofilm bactérien » ou « destruction du biofilm bactérien », on entend ici que les bactéries formant la communauté constituant le biofilm bactérien se séparent les unes des autres et de la surface, de sorte que le biofilm diminue voire disparait, rendant typiquement les bactéries à nouveau accessibles aux antibiotiques.

Dans un mode de réalisation particulier, ledit biofilm bactérien est dispersé à au moins 50%, en particulier au moins 55%, en particulier au moins 60%, en particulier au moins 65%, en particulier à au moins 70%, au moins 75%, au moins 80%, au moins 85%, au moins 90%, au moins 95% ou au moins 100%.

Avantageusement, ledit peptide natriurétique, en particulier ledit peptide ANP, propeptide d'ANP ou dérivé de peptide ANP, disperse le biofilm bactérien sans tuer les bactéries constituant ce biofilm.

Il est toutefois à noter que lorsque ledit peptide natriurétique, en particulier ledit peptide ANP, propeptide d'ANP ou dérivé de peptide ANP, est utilisé en combinaison avec un antibiotique, les bactéries constituant le biofilm peuvent être tuées par ledit antibiotique, notamment lorsqu'elles sont libérées suite à l'effet dispersif du peptide natriurétique, en particulier de l'ANP, d'un propeptide d'ANP ou d'un dérivé d'ANP.

*Application thérapeutique*

La présente invention concerne un peptide natriurétique choisi dans le groupe constitué de (i) l'ostéocrine, un propeptide d'ostéocrine ou dérivé d'ostéocrine, (ii) une lébétine (de préférence la lébétine 2α ou la lébétine 1 β), un fragment de lébétine ou un dérivé de lébétine ou d'un fragment de lébétine, et (iii) un peptide ANP, propeptide d'ANP ou dérivé de peptide ANP, dans laquelle le dérivé d'ostéocrine est une ostéocrine dans laquelle un ou plusieurs résidus d'acide aminé a été modifié chimiquement ou un variant peptidique d'ostéocrine, dans laquelle le dérivé de lébétine ou d'un fragment de lébétine est une lébétine ou un fragment de lébétine dans laquelle/lequel un ou plusieurs résidus d'acide aminé a été modifié chimiquement ou un variant peptidique de lébétine ou de fragment de lébétine, et dans laquelle ledit dérivé de peptide ANP est une forme tronquée d'ANP correspondant aux acides aminés 4 ou 5 à 28 de la forme entière de l'ANP, une forme ayant une structure qui est un dimère antiparallèle de l'ANP, un polymère constitué de monomères d'ANP, un peptide ANP dans lequel 1 à 4 acides aminés ont été supprimés, substitués ou ajoutés, un peptide ANP dans lequel a été substitué l'acide aminé Phe de la boucle en position 8, un peptide ANP dans lequel a été substitué l'acide aminé Phe en position 26 ou l'acide aminé Ser en position 25, l'urodilatine, le fsANP ou une version linéarisée de l'ANP avec clivage du pont disulfure pour son utilisation dans une méthode de traitement thérapeutique d'une infection bactérienne associée à un biofilm bactérien, telle que définie dans la section *«Biofilm bactérien et infection bactérienne »* ci-dessus chez un sujet, dans laquelle ledit peptide natriurétique disperse ledit biofilm bactérien.

Par « sujet », on entend ici un humain ou un animal tel qu'un mammifère non humain, un oiseau ou un poisson.

Dans un mode de réalisation particulier, le sujet souffre d'une pathologie respiratoire chronique, telle que la mucoviscidose, une bronchopneumopathie chronique obstructive ou une bronchectasie. De préférence, le sujet souffre de mucoviscidose.

Dans un autre mode de réalisation particulier, ledit sujet souffre de brûlures importantes (grand brûlé) ou d'une infection de surface.

Dans un autre mode de réalisation, ledit sujet porte un implant et/ou une prothèse.

Dans un mode de réalisation particulier, ledit peptide natriurétique, en particulier ledit peptide ANP, propeptide d'ANP ou dérivé de peptide ANP, est utilisé en combinaison avec un antibiotique.

Par « antibiotique », on entend ici une substance naturelle ou synthétique détruisant ou bloquant la croissance des bactéries.

Les antibiotiques incluent :

- les antibiotiques inhibiteurs de la synthèse des enveloppes bactériennes parmi lesquels :

   ○ les beta-lactamines, en particulier :

      ▪ les pénicillines dont les pénicillines du groupe A telles que l'amoxicilline ; les pénicillines du groupe G et V telles que la benzathine benzylpénicilline, la benzathine pénicilline, la benzathine phenoxyméthylpénicilline, les pénicillines G et la pénicilline V ; les pénicillines du groupe M telles que la cloxacilline et l'oxacilline ; les carboxypénicillines telles que la ticarcilline ; les uréidopénicillines telles que la pipéracilline ; les aminidopénicillines telles que le pivmécillinam ; et la témocilline ;
      ▪ les carbapénèmes telles que l'ertapénem, l'imipénem et le méropénem ;
      ▪ les monobactames tels que l'aztréonam ;
      ▪ les céphalosporines dont les céphalosporines de 1^ère génération telles que le céfaclor, le céfadroxil, la céfalexine, la céfalotine, la céfazoline et la céfradine ; les céphalosporines de 2^ème génération telles que le céfamandole, la céfoxitine, le céfuroxime sodique et le céfuroxime axétil ; et les céphalosporines de 3ème génération telles que le céfixime, le cefpodoxime proxétil, le céfodiam hexétil, le céfépime, le céfotaxime, le cefpirome, le ceftazidime et le ceftriaxone ;

- ∘ les fosfomycines telles que la fosfomycine et la fosfomycine trométamol ;
- ∘ les glycopeptides tels que la teicoplanine et la vancomycine ;
- ∘ les lipopeptides tels que la daptomycine ; et
- ∘ les polymyxines telles que la polymyxine B et la polymyxine E ou colistine ;

- les antibiotiques inhibiteurs de la synthèse des protéines, parmi lesquels :

- ∘ les aminosides tels que l'amikacine sulfate, la gentamicine, la néomycine, la nétilmycine, la spectinomycine, la streptomycine et la tobramycine ;
- ∘ les macrolides et apparentés, en particulier :

  - les macrolides vrais tels que l'amphotéricine B, l'azithromycine, la clarithromycine, l'érythromycine, la josamycine, la midécamycine la roxithromycine ;
  - les lincosamides tels que la clindamycine et la lincomycine ;
  - les kétolides tels que la télithromycine ; et
  - les synergistines telles que la pristinamycine ;

- ∘ les phénicoles tels que le thiamphénicol ;
- ∘ les cyclines telles que la chlortétracycline, la doxycycline, la lymécycline, la méthylènecycline, la minocycline et la tigécycline ;
- ∘ les acides fusidiques tels que l'acide fusidique ; et
- ∘ les oxazolidinones tels que le linézolide et le tedizolid ;

- les antibiotiques inhibiteurs de la synthèse des acides nucléiques, parmi lesquels :

- ∘ les quinolones telles que l'acide pipémidique, la fluméquine, l'énoxacine, la loméfloxacine, la norfloxacine, la ciprofloxacine, l'ofloxacine, la péfloxacine, la lévofloxacine et la moxifloxacine ;
- ∘ les quinoléines telles que l'hydroxyquinoléine ;
- ∘ la mupirocine ; et
- ∘ la rifamycine ;

- les antibiotiques inhibiteurs de la synthèse de l'acide folique parmi lesquels les sulfamides telles que la sulfadiazine, le sulfaméthizol, le sulfafueazole et le sulfaméthoxazole ; et
- les antibiotiques de mécanismes complexes ou méconnus parmi lesquels :

- ∘ les produits nitrés, en particulier :

  - les nitrofuranes tels que le nitrofurantoïne et le nifuroxazide ; et
  - les nitro-imidazoles tels que le métronidazole, l'omidazole et le tinidazole ; et

- ∘ l'éthambutol, l'isoniazide, la pyrazinamide, la rifabutine et la rifampicine.

Dans un mode de réalisation particulier, ledit antibiotique est un antibiotique inhibiteur de la synthèse protéique, en particulier un aminoside tel que la tobramycine ; un antibiotique inhibiteur de la synthèse d'acide nucléique, en particulier une quinolone telle que la ciprofloxacine ; un antibiotique inhibiteur de la synthèse des enveloppes bactériennes, en particulier une béta-lactamine, plus particulièrement un carbapénème tel que l'imipénem, le doripenem ou le meropenem ou un antibiotique agissant sur la structure de l'enveloppe bactérienne en particulier une polymyxine telle que la polymyxine B ou la colistine.

Dans un mode de réalisation particulier, ledit antibiotique est un antibiotique aminoside, une quinolone, un carbapénème ou une polymyxine. Plus particulièrement, ledit antibiotique peut être un antibiotique aminoside ou une quinolone.

Dans un autre mode de réalisation particulier, ledit antibiotique est la tobramycine, la ciprofloxacine, l'imipénem et/ou la colistine.

Plus particulièrement, ledit antibiotique peut être la tobramycine et/ou la ciprofloxacine.

Les inventeurs ont montré que l'utilisation combinée du peptide ANP avec ces antibiotiques présente un effet synergique sur la destruction des biofilms. Sans être lié par la théorie, il est supposé que le peptide ANP, en dispersant les bactériens constituant le biofilm, les rend plus accessibles aux antibiotiques, qui sont ainsi plus efficaces.

Dans un autre mode de réalisation particulier, ledit peptide natriurétique est utilisé en combinaison avec au moins un autre peptide natriurétique tel que défini dans la section *« Peptides natriurétiques ».*

Ainsi, dans un mode de réalisation particulier, lorsque ledit peptide natriurétique est l'ANP, un propeptide d'ANP ou dérivé de peptide ANP, il peut être utilisé en combinaison avec l'ostéocrine, propeptide d'ostéocrine ou dérivé d'ostéocrine, et/ou avec une lébétine (de préférence la lébétine 2$\alpha$ ou la lébétine 1$\beta$), un fragment de lébétine ou un dérivé de lébétine ou d'un fragment de lébétine.

Alternativement, dans un autre mode de réalisation particulier, lorsque ledit peptide natriurétique est l'ostéocrine, un propeptide d'ostéocrine ou dérivé d'ostéocrine, il peut être utilisé en combinaison avec l'ANP, propeptide d'ANP ou dérivé de peptide ANP et/ou avec une lébétine (de préférence la lébétine 2$\alpha$ ou la lébétine 1$\beta$), un fragment de lébétine ou un dérivé de lébétine ou d'un fragment de lébétine.

Alternativement, dans un autre mode de réalisation particulier, lorsque ledit peptide natriurétique est la lébétine (de préférence la lébétine 2$\alpha$ ou la lébétine 1$\beta$), un fragment de lébétine ou un dérivé de lébétine ou d'un fragment de lébétine, il peut être utilisé en combinaison avec l'ANP, propeptide d'ANP ou dérivé de peptide ANP et/ou avec l'ostéocrine, propeptide d'ostéocrine ou dérivé d'ostéocrine.

L'utilisation combinée de plusieurs peptides natriurétiques a l'avantage de pouvoir utiliser des doses plus faibles de chacun des composés, limitant ainsi encore plus efficacement les éventuels effets secondaires.

Dans ce mode de réalisation particulier, ledit peptide natriurétique peut en outre est utilisé en combinaison avec un antibiotique comme décrit ci-dessus, éventuellement de façon séquentielle (le dit peptide natriurétique d'abord, puis l'antibiotique).

Dans un mode de réalisation particulier, ledit peptide natriurétique, en particulier ledit peptide ANP, propeptide d'ANP ou dérivé de peptide ANP, est utilisé en combinaison avec toute autre substance adaptée pour le traitement du sujet souffrant de l'infection bactérienne tel que décrit ci-dessus, éventuellement de façon séquentielle (le dit peptide natriurétique d'abord, puis la substance adaptée).

En particulier, lorsque le sujet souffre d'une pathologie respiratoire chronique, ledit peptide natriurétique, en particulier ledit peptide ANP, propeptide d'ANP ou dérivé de peptide ANP, est de préférence utilisé en combinaison avec une substance adaptée pour le traitement de cette pathologie respiratoire chronique, éventuellement de façon séquentielle (le dit peptide natriurétique d'abord, puis la substance adaptée).

Ainsi, dans le mode de réalisation dans lequel ledit sujet souffre de mucoviscidose, ledit peptide natriurétique, en particulier ledit peptide ANP, propeptide d'ANP ou dérivé de peptide ANP, est de préférence utilisé en combinaison avec une substance pour la clairance muco-ciliaire, telle que la Dnase (par exemple le Pulmozyme®), et/ou bronchodilatatrice, éventuellement de façon séquentielle (une substance pour la clairance muco-ciliaire d'abord, puis le dit peptide natriurétique).

Dans ces modes de réalisation particuliers, ledit peptide natriurétique peut en outre être utilisé en combinaison avec un antibiotique comme décrit ci-dessus et/ou avec un autre peptide natriurétique comme décrit ci-dessus, de préférence avec un antibiotique comme décrit ci-dessus.

Dans le contexte de l'invention, le terme « traitement » ou « traiter » signifie inverser, soulager ou inhiber le progrès de la maladie à laquelle ce terme s'applique, ou un ou plusieurs symptômes de cette maladie.

Par « quantité thérapeutiquement efficace », on entend ici une quantité suffisante du composé d'intérêt pour disperser le biofilm bactérien dans le traitement de l'infection bactérienne, à un ratio risque/bénéfice raisonnable applicable à n'importe quel traitement médical. Il sera bien compris cependant que l'utilisation journalière totale des composés utilisés dans le cadre de l'invention sera décidée par le médecin traitant dans le cadre de son jugement médical. Le niveau de dose thérapeutiquement efficace spécifique à un sujet particulier dépendra d'un certain nombre de facteurs dont la maladie à traiter et la gravité de cette maladie, l'activité des composés spécifiquement employés, l'âge, le poids, l'état de santé général, le sexe et le régime alimentaire, le temps d'administration, la voie d'administration et le vitesse d'excrétion des composés spécifiques employés, la durée du traitement, les médicaments utilisés en combinaison ou de manière coïncidente avec les composés spécifiques employés, et des facteurs semblables bien connus dans le domaine médical. Par exemple, il est bien connu dans le domaine de commencer les doses de composés à des niveaux plus faibles que ceux requis pour atteindre l'effet thérapeutique désiré et d'augmenter graduellement le dosage jusqu'à ce que l'effet désiré soit obtenu.

Les composés utilisés dans le contexte de l'invention peuvent être administrés sous la forme d'une composition pharmaceutique comprenant des excipients pharmaceutiquement acceptables, et éventuellement des matrices à libération différée, telles que des polymères biodégradables, pour former des compositions thérapeutiques.

Par « pharmaceutique » ou « pharmaceutiquement acceptable », on entend ici des entités moléculaires et des compositions qui ne produisent pas de réactions secondaires, allergiques ou autrement fâcheuse quand elles sont administrées à un mammifère, en particulier à un humain. Un véhicule ou excipient pharmaceutiquement acceptable désigne une charge non-toxique solide, semi-solide ou liquide, un diluant, une matière d'encapsulation ou un auxiliaire de formulation de tout type.

La forme des compositions pharmaceutiques comprenant les composés utilisés dans le contexte de l'invention et la voie d'administration dépendront naturellement de la maladie à traiter, de la sévérité de la maladie, de l'âge, du poids et du genre du patient, etc...

Les composés utilisés dans le contexte de l'invention peuvent être formulés pour une administration par voie aérienne ou inhalation (en particulier par nébulisation), orale, parentéral, intranasale, intraveineuse, intramusculaire, topique, sous-cutanée ou intraoculaire par exemple.

Dans un mode de réalisation particulier, les composés utilisés dans le contexte de l'invention sont administration par voie aérienne ou inhalation (en particulier par nébulisation).

Dans un mode de réalisation particulier, le peptide natriurétique, en particulier le peptide ANP (ou propeptide ou dérivé), utilisé dans le contexte de l'invention est administré à une dose journalière de 0.3 ng/ml à 3.000 ng/ml.

Dans un autre mode de réalisation particulier, l'antibiotique utilisé en combinaison est administré à une dose journalière maximale de 600 mg (deux fois 300 mg par jour).

*Composition pharmaceutique et combinaison pharmaceutique*

La présente invention concerne également une composition pharmaceutique comprenant (A) un peptide natriurétique choisi dans le groupe constitué d'une lébétine (de préférence la lébétine $2\alpha$ ou la lébétine $1\beta$), un fragment de lébétine ou un dérivé de lébétine ou d'un fragment de lébétine et (B) un antibiotique tel que défini dans la section *« Application thérapeutique »* ci-dessus, dans laquelle le dérivé de lébétine ou d'un fragment de lébétine est une lébétine ou un fragment de lébétine dans laquelle/lequel un ou plusieurs résidus d'acide aminé a été modifié chimiquement ou un variant peptidique.

Dans un mode de réalisation particulier, la composition selon l'invention comprend en outre un véhicule ou excipient pharmaceutiquement acceptable tel que défini ci-dessus.

Dans un autre mode de réalisation particulier, la composition selon l'invention comprend en outre un autre peptide natriurétique tel que défini dans la section « *Peptides natriurétiques* » ci-dessus.

La présente invention concerne également une combinaison pharmaceutique antibactérienne comprenant (A) un peptide natriurétique choisi dans le groupe constitué de (i) l'ostéocrine, propeptide d'ostéocrine ou dérivé d'ostéocrine, (ii) une lébétine (de préférence la lébétine $2\alpha$ ou la lébétine $1\beta$), un fragment de lébétine ou un dérivé de lébétine ou d'un fragment de lébétine et (iii) un peptide ANP, propeptide d'ANP ou dérivé de peptide ANP tel que défini dans la section « *Peptides natriurétiques* » ci-dessus et (B) un antibiotique tel que défini dans la section *« Application thérapeutique »* ci-dessus pour une utilisation simultanée, séparée ou séquentielle dans le traitement thérapeutique chez un sujet tel que défini ci-dessus d'une infection bactérienne associée à un biofilm bactérien telle que définie dans la section *« Biofilm bactérien et infection bactérienne »* ci-dessus, dans laquelle ledit peptide natriurétique disperse ledit biofilm bactérien,

dans laquelle le dérivé d'ostéocrine est une ostéocrine dans laquelle un ou plusieurs résidus d'acide aminé a été modifié chimiquement ou un variant peptidique d'ostéocrine,
dans laquelle le dérivé de lébétine ou d'un fragment de lébétine est une lébétine ou un fragment de lébétine dans laquelle/lequel un ou plusieurs résidus d'acide aminé a été modifié chimiquement ou un variant peptidique de lébétine ou de fragment de lébétine, et
dans laquelle ledit dérivé de peptide ANP est une forme tronquée d'ANP correspondant aux acides aminés 4 ou 5 à 28 de la forme entière de l'ANP, une forme ayant une structure qui est un dimère antiparallèle de l'ANP, un polymère constitué de monomères d'ANP, un peptide ANP dans lequel 1 à 4 acides aminés ont été supprimés, substitués ou ajoutés, un peptide ANP dans lequel a été substitué l'acide aminé Phe de la boucle en position 8, un peptide ANP dans lequel a été substitué l'acide aminé Phe en position 26 ou l'acide aminé Ser en position 25, l'urodilatine, le fsANP ou une version linéarisée de l'ANP avec clivage du pont disulfure.

Dans le contexte de l'invention, le terme « combinaison » ou « combinaison pharmaceutique » définit soit une combinaison fixée dans une seule forme d'unité de dosage, soit un kit pour administration combinée dans lequel le peptide natriurétique et l'antibiotique peuvent être administrés indépendamment au même moment ou séparément en des intervalles de temps qui permettent aux partenaires de la combinaison de montrer un effet synergique.

Les composés de la combinaison de l'invention peuvent donc être formulés en une ou deux combinaisons pharmaceutiques séparées, chaque composition étant pour la même voie d'administration ou pour des voies d'administration différentes.

Dans un mode de réalisation particulier, ladite combinaison pharmaceutique selon l'invention comprend en outre (C) un autre peptide natriurétique tel que défini dans la section « *« Peptides natriurétiques »,* et/ou (D) une autre substance adaptée pour le traitement du sujet souffrant de l'infection bactérienne tel que défini dans la section *« Application*

*thérapeutique »* ci-dessus.

Dans ce mode de réalisation particulier, la combinaison peut être une combinaison fixée dans une seule forme d'unité de dosage, ou un kit pour une administration combinée dans lequel le peptide natriurétique, l'antibiotique, l'autre peptide natriurétique et/ou l'autre substance adaptée pour le traitement du sujet, peuvent être administrée indépendamment au même moment ou séparément en des intervalles de temps qui permettent aux partenaires de la combinaison de montrer un effet synergique.

Dans ce mode de réalisation, les composés de la combinaison peuvent donc être formulés en une, deux, trois ou quatre combinaisons pharmaceutiques séparées, chaque composition étant pour la même voie d'administration ou pour des voies d'administration différentes.

Pour préparer les composés pharmaceutiques utilisées dans le cadre de l'invention, une quantité efficace des composés utilisées dans le cadre de l'invention peut être dissoute ou dispersée dans un véhicule ou un milieu aqueux pharmaceutiquement acceptable.

Les formes pharmaceutiques adaptées à une utilisation injectable incluent les solutions ou dispersions aqueuses stériles et les poudres stériles pour une préparation extemporanée de solutions ou dispersions injectables stériles, par exemple sous forme micronisée. Dans tous les cas, la forme doit être stérile et doit être fluide de manière à ce que l'utilisation par seringue soit facile. Elle doit être stable sous les conditions de fabrication et de conservation et doit être préservée contre l'action contaminante de microorganismes tels que les bactéries, les virus ou les champignons.

Le véhicule peut être un solvant ou un milieu de dispersion comprenant, par exemple, de l'eau, de l'éthanol, un polyol (par exemple, du glycérol, du propylène glycol, du polyéthylène glycol liquide, ou autre), et mélanges appropriés de ceux-ci. Une fluidité appropriée peut être maintenue par exemple en utilisant un revêtement telle que de la lécithine, en maintenant une taille de particule requise dans le cas d'une dispersion, et en utilisant des tensioactifs, des agents stabilisants, des cryoprotecteurs ou des anti-oxydants. La prévention de l'action des microorganismes peut être apportée par des agents antibactériens et antifongiques. Dans de nombreux cas, il sera préférable d'inclure des agents isotoniques, par exemple des sucres ou du chlorure de sodium.

Les solutions injectables stériles peuvent être préparées en incorporant les composés actifs dans une quantité requise du solvant approprié avec plusieurs des autres ingrédients, suivi d'une stérilisation filtrée. En général, les dispersions sont préparées en incorporant les divers ingrédients actifs stérilisés dans un véhicule stérile qui contient le milieu de dispersion basique et les autres ingrédients requis. Dans le cas des poudres stériles pour la préparation de solutions injectables stériles, les méthodes préférées de préparation sont les techniques de séchage sous vide et de lyophilisation, qui produisent une poudre de l'ingrédient actif plus n'importe quel ingrédient additionnel désiré à partir d'une solution de ceux-ci préalablement stérilisée par filtration.

Pour une administration parentérale dans une solution aqueuse, par exemple, la solution sera de préférence tamponnée de manière appropriée si nécessaire et le diluent liquide rendu isotonique avec une solution saline ou de glucose suffisante. Ces solutions aqueuses particulières sont particulièrement appropriées pour une administration intraveineuse, intramusculaire, sous-cutanée et intrapéritonéale.

Les formulations de compositions pharmaceutiques pour une administration par inhalation sont bien connues de l'homme du métier. En général, les ingrédients actifs sont délivrés sous la forme d'un spray aérosol à partir d'un inhalateur pressurisé à dose mesurée en utilisant un gaz propulseur approprié, tel que le dichlorodifluorométhane, le trichlorofluorométhane, le dichloro-tétrafluoroéthane ou le dioxyde carbone, sous la forme de poudre administrée en utilisant un inhalateur à poudre sèche, ou, sous la forme d'un aérosol à liquide aqueux en utilisant un nébuliseur. Les nébuliseurs pour délivrance d'un aérosol liquide peuvent être catégorisés en nébuliseurs à jet mis en œuvre par un flux pressurisé d'air en utilisant un compresseur portable ou un fournisseur d'air central à l'hôpital, les nébuliseurs à ultrason incorporant un piezo-cristal pour fournir l'énergie pour générer l'aérosol hors d'une fontaine ultrasonique, et des nébuliseurs électroniques basés sur le principe d'une membrane vibrante perforée.

*Utilisations non-thérapeutiques*

La présente invention concerne également l'utilisation *in vitro* ou *ex vivo* d'un peptide natriurétique choisi dans le groupe constitué de (i) l'ostéocrine, un propeptide d'ostéocrine ou dérivé d'ostéocrine, (ii) une lébétine (de préférence la lébétine 2α ou la lébétine 1β), un fragment de lébétine ou un dérivé de lébétine ou d'un fragment de lébétine et (iii) un peptide ANP, un propeptide d'ANP ou un dérivé de peptide ANP, pour disperser un biofilm bactérien, dans laquelle le dérivé d'ostéocrine est une ostéocrine dans laquelle un ou plusieurs résidus d'acide aminé a été modifié chimiquement ou un variant peptidique d'ostéocrine,

dans laquelle le dérivé de lébétine ou d'un fragment de lébétine est une lébétine ou un fragment de lébétine dans laquelle/lequel un ou plusieurs résidus d'acide aminé a été modifié chimiquement ou un variant peptidique de lébétine ou de fragment de lébétine, et

dans laquelle ledit dérivé de peptide ANP est une forme tronquée d'ANP correspondant aux acides aminés 4 ou 5 à 28 de la forme entière de l'ANP, une forme ayant une structure qui est un dimère antiparallèle de l'ANP, un polymère constitué de monomères d'ANP, un peptide ANP dans lequel 1 à 4 acides aminés ont été supprimés, substitués ou ajoutés, un peptide ANP dans lequel a été substitué l'acide aminé Phe de la boucle en position 8, un peptide ANP dans lequel a été substitué l'acide aminé Phe en position 26 ou l'acide aminé Ser en position 25, l'urodilatine, le fsANP ou une version linéarisée de l'ANP avec clivage du pont disulfure.

Le peptide natriurétique, en particulier l'ANP, propeptide d'ANP ou dérivé de peptide ANP peut ainsi typiquement être utilisé dans des applications antisalissures (« *antifouling* »)*,* par exemple sur les bateaux ou dans les canalisations.

La présente divulgation décrit ainsi également une méthode, non couverte par les revendications, *in vitro* ou *ex vivo* de dispersion d'un biofilm bactérien sur une surface, comprenant l'application sur ladite surface d'une composition comprenant un peptide natriurétique choisi dans le groupe constitué de (i) l'ostéocine, propeptide d'ostéocrine ou dérivé d'ostéocrine, (ii) une lébétine (de préférence la lébétine 2α ou la lébétine 1β), un fragment de lébétine ou un dérivé de lébétine ou d'un fragment de lébétine, et (iii) un peptide ANP, un propeptide d'ANP ou un dérivé de peptide ANP, tel que défini dans la section *« Peptides natriurétiques »* ci-dessus.

Ladite surface peut typiquement être la surface d'une canalisation, la surface d'un bateau, la surface d'un dispositif implantable avant implantation.

L'application de ladite composition sur ladite surface peut être mise en œuvre par toute technique appropriée, dépendant de la formulation de la composition.

La présente invention sera décrite plus en détail par les exemples ci-dessous et figures ci-dessous.

## Brève description des séquences

| SEQ ID NO | Description | Séquence |
|---|---|---|
| 1 | Séquence d'acides aminés de l'ANP | SLRRSSCFGGRMDRIGAQSGLGCNS FRY |
| 2 | Séquence d'acides aminés de l'urodilatine | TAPRSLRRSSCFGGRMDRIGAQSGL GCNSFRY |
| 3 | Séquence d'acides aminés du proANP | NPMYNAVSNADLMDFKNLLDHLEEK MPLEDEVVPPQVLSEPNEEAGAALSP LPEVPPWTGEVSPAQRDGGALGRGP WDSSDRSALLKSKLRALLTAPRSLRR SSCFGGRMDRIGAQSGLGCNSFRY |
| 4 | Séquence d'acides aminés du preproANP | MSSFSTTTVSFLLLLAFQLLGQTRAN PMYNAVSNADLMDFKNLLDHLEEKM PLEDEVVPPQVLSEPNEEAGAALSPL PEVPPWTGEVSPAQRDGGALGRGP WDSSDRSALLKSKLRALLTAPRSLRR SSCFGGRMDRIGAQSGLGCNSFRY |
| 5 | Séquence d'acides aminés de l'ostéocrine | SFSGFGSPLDRLSAGSVDHKGKQRK VVDHPKRRFGIPMDRIGRNRLSNSR |
| 6 | Séquence d'acides aminés de la pro-ostéocrine | VDVTTTEAFDSGVIDVQSTPTVREEK SATDLTAKLLLLDELVSLENDVIETKK KRSFSGFGSPLDRLSAGSVDHKGKQ RKVVDHPKRRFGIPMDRIGRNRLSNS RG |

(suite)

| SEQ ID NO | Description | Séquence |
|---|---|---|
| 7 | Séquence d'acides aminés de la prépro-ostéocrine | MLDWRLASAHFILAVTLTLWSSGKVL SVDVTTTEAFDSGVIDVQSTPTVREE KSATDLTAKLLLLDELVSLENDVIETK KKRSFSGFGSPLDRLSAGSVDHKGK QRKVVDHPKRRFGIPMDRIGRNRLSN SRG |
| 8 | Séquence d'acides aminés de la lébétine 2α | GDNKPPKKGPPNGCFGHKIDRIGSHS GLGCNKVDDNKG |
| 9 | Séquence d'acides aminés de la lébétine 1β | DNKPPKKGPPNG |

**Brève description des figures**

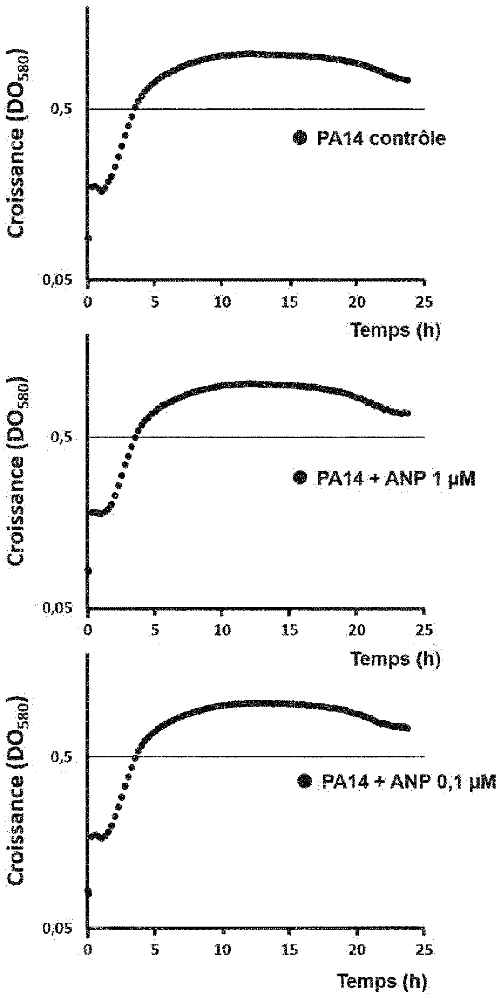

La **Figure 1** montre des graphiques représentant la croissance de *P. aeruginosa* en présence de différentes concentrations d'ANP.

**Exemples**

**Exemple 1** : **Effet de l'ANP seul (0,1 μM) sur les biofilms en formation, sur les biofilms préformés de *P. aeruginosa* et sur la croissance de *P. aeruginosa*.**

Cet exemple montre l'action de l'ANP sur les biofilms préformés de *P. aeruginosa.*

Matériel et méthodes

*Substances testées, souches bactériennes et cultures bactériennes*

La souche de *P. aeruginosa* sauvage PA14 utilisée était de la Havard Medical School (Noston, MA) (Liberati et al. (2006) Proc. Natl. Acad. Sci. USA 103 :2833-2838).

Les souches bactériennes ont été cultivées à 37°C dans du milieu Luria Bertani (LB) sous agitation.

*Formation du biofilm de P. aeruginosa en conditions dynamiques*

Après 3 h de pré-culture en milieu LB à 37°C, *P. aeruginosa* a été inoculée à une $DO_{600}$ = 0,08 dans du milieu LB et sous-cultivé pendant 2 h.

L'ANP (Calbiochem Merck) a été ajouté 2 h après le début de la culture, un moment correspondant au milieu de la phase exponentielle de croissance des bactéries. La densité finale bactérienne et l'absence de contamination ont été contrôlées par ensemencement

Les biofilms ont été formés en conditions dynamiques à 37°C dans une chambre à flux à 3 canaux comme décrit dans Bazire et al. (2010) J. Bacteriol. 192 :3001-3010. Brièvement, à partir d'une pré-culture de 18h, une suspension bactérienne à $DO_{600}$=0,08 préparée dans le l'eau physiologique stérile a été injectée dans chaque canal de la chambre à flux. Les bactéries ont été laissées pendant 2h à 37°C en conditions statiques (sans flux) afin que celles-ci adhèrent à la lame de verre. L'étude de la formation de biofilm a ensuite été réalisée sous un flux de milieu LB (2,5 ml/h) pendant 24h à 37°C.

*Destruction du biofilm de P. aeruginosa en conditions dynamiques*

Pour étudier l'impact de l'ANP sur les biofilms préformés pendant 24 h, 300 μl d'ANP (0.1 μM) ou 300 μl d'eau ultra-pure stérile (condition contrôle) ont été injectés dans différents canaux de la chambre à flux. Le traitement du biofilm préformé a été réalisé pendant 2 h à 37°C en conditions statiques. Après les 2h, le flux de milieu a été de nouveau appliqué pendant 15 min afin d'éliminer les cellules qui se seraient détachées du biofilm sous l'action du traitement.

Les biofilms ont été observés en microscopie confocale à balayage laser (Zeiss LSM710 (Zeiss) après avoir été marqués pendant 15 min avec 5 μM de fluorochrome Syto9 green (Invitrogen). Des prises de vue ont été réalisées dans les différentes couches du biofilm permettant ainsi une reconstitution tridimensionnelle ; au minimum 3 prises de vue ont été réalisées en des points différents d'un même canal de la chambre à flux. Les fichiers de chaque prise de vue ont été ensuite analysés par le logiciel COMSTAT (Heydorn et al. (2000) Microbiology 146 :2395-2407), 3 prises de vue d'au moins 3 expériences indépendantes sont analysés, permettant de déterminer les épaisseurs moyennes et maximales des biofilms ainsi que le biovolume bactérien.

*Culture cellulaire*

La lignée de cellules épithéliales pulmonaires humaines de type II A549 (ATCC-CCL185TM, ATCC Manassas, VA) a été cultivée à 37°C dans une atmosphère à 5% $CO_2$ dans un milieu DMEM (Lonza) complémenté avec 10% de sérum de veau fœtal (Lonza) et 1% de pénicilline et streptomycine (Penistrep, Lonza). En routine, les cellules ont été ensemencées dans une fiole de 25 ml et utilisées à 80% de confluence.
Pour les tests de cytotoxicité, les cellules ont été ensemencées dans des plaques 24 puits à une densité finale de $3 \times 10^5$ cellules par puits, et cultivées pendant 48 h avant utilisation. Un minimum de 24 h avant les tests d'infection, les cellules ont été déprivées en antibiotiques et en sérum de veau fœtal par ajout d'un milieu sans sérum frais.

*Mesure de la libération de lactate déshydrogénase cytosolique (LDH) par les cellules A549*

La LDH est une enzyme cytosolique stable libérée dans le milieu de culture après lyse cellulaire et donc un marqueur de mort cellulaire. La quantité de LDH libérée par les cellules eucaryotes en présence de bactéries, exposées ou non à l'ANP (concentration de 1 μM à 10 nM), a été déterminée en utilisant le test enzymatique Cytotox 96 (Promega, Charbonnières, France). Les cellules A549 ont été incubées pendant 6 h avec un contrôle (sans traitement) ou avec *P. aeruginosa* PA14 (pré-traitées à l'ANP) à une multiplicité d'infection de 10. Un tampon de lyse, consistant en une solution de Triton X-100 (9% dans l'eau), a été employé pour déterminer le maximum de LDH potentiellement libéré par les cellules A549 dans les conditions expérimentales (100% de libération de LDH). Un niveau bruit de fond a été établi en utilisant le milieu de culture seul, et défini comme 0% de libération de LDH, pour éliminer la contribution du milieu de culture. Le pourcentage de libération de LDH dans la population cellulaire a ensuite a été calculé en utilisant l'équation :

$$\%LDH = \frac{DO\ \acute{e}chantillon}{DO\ 100\%} \times 100$$

Le test était suffisamment sensible pour mesurer une concentration de LDH équivalente à une lyse de 1% de la population cellulaire.

Résultats

*Effet de l'ANP sur un biofilm préformé de P. aeruginosa*

Les inventeurs ont étudié le potentiel d'activité anti-biofilm de l'ANP sur un biofilm établi de *P. aeruginosa*. Dans cette optique, après 24 h de formation d'un biofilm de *P. aeruginosa* dans un système d'écoulement dynamique, les inventeurs ont exposé pendant 2 h les bactéries à l'ANP à 0.1 μM. Dans ces conditions, les inventeurs ont observé une dispersion du biofilm qui a été perturbée par l'ANP, et les structures en forme de champignon, observées en condition contrôle et caractéristique d'un biofilm de *P. aeruginosa*, étaient absentes après exposition à l'ANP. La biomasse bactérienne a été réduite après exposition à l'ANP pendant 2 heures à 0,1 μM de 81,4 ± 4,1% (P < 0.001) par rapport à la biomasse présente dans la condition contrôle. En parallèle, les inventeurs ont observé une réduction importante de l'épaisseur du biofilm après 2 h d'exposition à l'ANP.
Des résultats similaires ont été obtenus lorsqu'on expose le biofilm pré-formé à l'ANP pendant 30 minutes. Dans ces conditions les inventeurs ont observé que le biofilm était fortement dispersé par l'ANP, et les structures en forme de champignon, observées en condition contrôle et caractéristiques d'un biofilm de *P. aeruginosa*, étaient absentes après exposition à l'ANP. La biomasse bactérienne restante a été réduite après exposition à l'ANP (30 minutes) de 80,2 ± 2,0% (P < 0.05) par rapport à la biomasse présente dans les conditions contrôles.

*Effet de l'ANP sur la croissance de P. aeruginosa*

Afin de déterminer un potentiel effet direct de l'ANP sur la croissance de P. *aeruginosa*, les inventeurs ont étudié l'impact de l'ANP à 1 μM et 0,1 μM sur la croissance de *P. aeruginosa* P14 dans un milieu liquide. Aucune des concentrations testées d'ANP n'a affecté la croissance bactérienne comme le montre la Figure 1.

Ainsi, l'effet de l'ANP sur les biofilms préformés ne peuvent pas être dus à une action sur la croissance de la bactérie

*Effet de l'ANP sur la cytotoxicité bactérienne vis-à-vis de cellules pulmonaires humaines en culture*

L'activité cytotoxique de *P. aeruginosa* exposée à différentes concentrations d'ANP (1 μM à 10 nM) a été étudiée en utilisant des cellules pulmonaires A549. Les inventeurs ont observé que l'ANP, à n'importe quelle concentration, n'avait pas d'impact sur l'activité cytotoxique de *P. aeruginosa* vis-à-vis des cellules pulmonaires, comme montré dans le tableau ci-dessous.

| Conditions expérimentales | % de LDH (cellules mortes) |
|---|---|
| **PA14 non traitées** | $56,4 \pm 5,5$ |
| **PA14 + ANP ($10^{-6}$ M)** | $58,9 \pm 5,8$ |
| **PA14 + ANP ($10^{-7}$ M)** | $53,5 \pm 5,3$ |
| **PA14 + ANP ($10^{-8}$ M)** | $54,5 \pm 5,5$ |

Conclusion

Les inventeurs ont ainsi montré dans cet exemple que l'ANP, utilisé à une concentration de 0,1 μM empêche totalement la formation du biofilm de *P. aeruginosa* et détruit fortement un biofilm préformé de *P. aeruginosa*.

Pendant longtemps, les études pour empêcher la formation des biofilms se sont concentrées sur les étapes initiales de la formation des biofilms, incluant l'adhésion et la maturation du biofilm. Les concentrations de drogues nécessaires pour inhiber la formation d'un biofilm sont généralement beaucoup plus faibles que celles nécessaires pour détruire ou perturber un biofilm préformé.

L'intérêt principal de l'ANP, ici mis en évidence par les inventeurs, est que son utilisation à 0,1 μM pendant 2 h voir 30 minutes, est suffisante pour disperser environ 80 % de la structure du biofilm. Cela est particulièrement intéressant, en particulier en vue d'une utilisation en synergie avec des antibiotiques.

Cet effet de l'ANP sur le biofilm de *P. aeruginosa* est plus important que l'effet d'inhibition de la formation observée précédemment avec le CNP et présente un intérêt supplémentaire car le CNP augmentait légèrement la virulence et la cytotoxicité bactérienne, en activant les systèmes du quorum-sensing bactériens.

Ici, les inventeurs ont démontré que l'ANP, malgré sa forte action anti-biofilm, ne tuait pas les bactéries et n'augmentait pas la cytotoxicité de *P. aeruginosa* vis-à-vis de cellules pulmonaires humaines en culture. Le fait de ne pas tuer les bactéries est particulièrement intéressant car cela évite l'émergence de souches résistantes.

**Exemple 2 : Effet dose-dépendant de l'ANP sur les biofilms préformés de P. *aeruginosa***

Cet exemple montre l'action de l'ANP seul sur les biofilms préformés de *P. aeruginosa* dépend de la dose utilisée et est visible dès 0,3 ng/ml.

Matériel et méthodes

Le matériel et méthodes utilisé dans cet exemple est identique à celui décrit ci-dessus dans l'exemple 1 *(Destruction du biofilm de P. aeruginosa en conditions dynamiques)*.

Résultats

Les inventeurs ont étudié le potentiel d'activité anti-biofilm de l'ANP sur un biofilm établi de *P. aeruginosa à* différentes concentrations d'ANP : 0,3 ng/ml, 3 ng/ml et 30 ng/ml et comparé avec celui présenté dans l'exemple 1 ([ANP] = 300 ng/ml ou 0,1 μM), après une exposition de 2 h ou 30 min.

Les inventeurs ont observé que le biofilm était fortement dispersé par l'ANP dès la concentration la plus faible de 0,3 ng/ml et dès une exposition de 30 min.

Les résultats obtenus sont décrits dans le tableau suivant.

| Conditions expérimentales | Biovolume (2h) ($\mu m^3/\mu m^2$) | % inhibition (2h) | Biovolume (30 min) ($\mu m^3/\mu m^2$) | % d'inhibition (30 min) |
|---|---|---|---|---|
| PA14 non traitées | 21,1 ¶ | | 22,19 | |
| | | | | |
| ANP (0,3 ng/ml) | 10,6 | 45,1 (**) | 9,5 | 57,5 (*) |
| ANP (3 ng/ml) | 6,3 | 65,9 (***) | 8,8 | 59 ,1 (*) |
| ANP (30 ng/ml) | 4,4 | 72,5 (***) | 6,7 | 69,0 (*) |
| ANP (300 ng/ml) | 4,9 | 81,4 (***) | 4,4 | 80,2 (*) |
| ¶ : Moyenne de tous les témoins. * : p < 0.05 *vs* biofilm non-traités ; ** : p < 0.01 vs biofilms non-traitées ; *** : p < 0.001 vs biofilms non-traitées. | | | | |

Cet exemple confirme donc l'intérêt de l'ANP, actif à très faible concentration, et dès 30 min d'exposition.

**Exemple 3** : **Effet de l'ostéocrine sur les biofilms préformés de *P. aeruginosa***

Cet exemple montre l'action de l'ostéocrine seul sur les biofilms préformés de *P. aeruginosa* à la dose de $10^{-8}$ M.

Matériel et méthodes

Le matériel et méthodes utilisé dans cet exemple est identique à celui décrit ci-dessus dans l'exemple 1 *(Destruction du biofilm de P. aeruginosa en conditions dynamiques).*

Résultats

Les inventeurs ont étudié le potentiel d'activité anti-biofilm de l'ostéocrine sur un biofilm établi de *P. aeruginosa à* la concentration de $10^{-8}$ M et après une exposition de 2 h.
Les inventeurs ont observé que le biofilm était fortement et significativement dispersé par l'ostéocrine.

| Conditions expérimentales | Biovolume (2h) ($\mu m^3/\mu m^2$) | % inhibition (2h) |
|---|---|---|
| PA14 non traitées (control) | 29,8 | |
| Osteocrine ($10^{-8}$ M) | 12,1 | 59,4 (***) |

**Exemple 4** : **Effet du peptide Lébétine 2$\alpha$ sur les biofilms préformés de *P. aeruginosa***

Cet exemple montre l'action du peptide lébétine L2 alpha seul sur les biofilms préformés de *P. aeruginosa à* la dose de $10^{-8}$ M.

Matériel et méthodes

Le matériel et méthodes utilisé dans cet exemple est identique à celui décrit ci-dessus dans l'exemple 1, *(Destruction du biofilm de P. aeruginosa en conditions dynamiques).*

Résultats

Les inventeurs ont étudié le potentiel d'activité anti-biofilm du peptide lébétine L2 alpha sur un biofilm établi de *P. aeruginosa à* la concentration de $10^{-8}$ M et après une exposition de 2 h.
Les inventeurs ont observé que le biofilm était fortement et significativement dispersé par le peptide lébétine L2 alpha.

| Conditions expérimentales | Biovolume (2h) ($\mu m^3/\mu m^2$) | % inhibition (2h) |
|---|---|---|
| PA14 non traitées (control) | 29,7 | |

(suite)

| Conditions expérimentales | Biovolume (2h) ($\mu m^3/\mu m^2$) | % inhibition (2h) |
|---|---|---|
| Lébétine L2alpha ($10^{-8}$ M) | 8,9 | 70,2 (***) |

## Exemple 5 : Effet du peptide Lébétine 1β sur les biofilms préformés de *P. aeruginosa*

Cet exemple montre l'action du peptide lébétine L1 bêta seul sur les biofilms préformés de *P. aeruginosa* à la dose de $10^{-8}$ M.

### Matériel et méthodes

Le matériel et méthodes utilisé dans cet exemple est identique à celui décrit ci-dessus dans l'exemple 1, (*Destruction du biofilm de P. aeruginosa en conditions dynamiques*).

### Résultats

Les inventeurs ont étudié le potentiel d'activité anti-biofilm du peptide lébétine L1 Bêta sur un biofilm établi de *P. aeruginosa* à la concentration de $10^{-8}$ M et après une exposition de 2 h.

Les inventeurs ont observé que le biofilm était fortement dispersé par le peptide lébétine L1 Bêta.

| Conditions expérimentales | Biovolume (2h) ($\mu m^3/\mu m^2$) | % inhibition (2h) |
|---|---|---|
| PA14 non traitées (control) | 21,0 | |
| Lébétine L1 Bêta ($10^{-8}$ M) | 6,9 | 67,0 |

## Exemple 6 : Effet d'une combinaison d'ANP et de tobramycine sur les biofilms préformés de *P. aeruginosa*

Cet exemple montre l'action synergique la combinaison ANP + tobramycine sur les biofilms préformés de *P. aeruginosa.*

### Matériel et méthodes

Le matériel et méthodes utilisé dans cet exemple est identique à celui décrit ci-dessus dans l'exemple 1, avec les précisions suivantes :

Pour étudier l'impact de l'ANP en combinaison avec la tobramycine sur des biofilms préformés de 24 h (comme décrit dans l'exemple 1), 300 $\mu$l d'un mélange d'ANP (à 1 nM) et de tobramycine (50 $\mu$g/ml) ou 300 $\mu$l de tobramycine seul (condition contrôle) ont été injectés dans différents canaux de la chambre à flux. Le traitement du biofilm préformé a été réalisé pendant 2 h à 37°C en conditions statiques. Après les 2 h, le flux de milieu a été de nouveau appliqué pendant 15 min afin d'éliminer les cellules qui se seraient détachées du biofilm sous l'action du traitement.

Les biofilms ont été observés en microscopie confocale à balayage laser comme décrit dans l'exemple 1. Pour tester l'intégrité membranaire des bactéries, un mélange de 5 $\mu$M de SYTO 9 green et 0,3 $\mu$M d'iodure de propidium (IP) a été utilisé (Live/Dead BacLight Bacterial Viability Kit, Invitrogen).

### Résultats

Les inventeurs ont étudié le potentiel d'activité anti-biofilm de la combinaison ANP (à $10^{-9}$ M ; 3 ng/ml) + tobramycine (à 50 $\mu$g/ml ou à 10 $\mu$g/ml) sur un biofilm établi de *P. aeruginosa.* Le biovolume du biofilm a été déterminé et les résultats sont décrits dans les tableaux ci-dessous.

| Conditions expérimentales | Biovolume ($\mu m^3/\mu m^2$) | % inhibition |
|---|---|---|
| PA14 non traitées | 13,5 | |
| Tobramycine (50 $\mu$g/ml) | 3,26 | 75,9 |
| ANP ($10^{-9}$ M) | 4,53 | 66,4 |

(suite)

| Conditions expérimentales | Biovolume ($\mu m^3/\mu m^2$) | % inhibition |
|---|---|---|
| Tobramycine (50 $\mu$g/ml) + ANP ($10^{-9}$ M) | 0,5 | 96,3 |

| Conditions expérimentales | Biovolume ($\mu m^3/\mu m^2$) | % inhibition |
|---|---|---|
| PA14 non traitées | 13,5 | |
| Tobramycine (10 $\mu$g/ml) | 2,9 | 78,5 |
| ANP ($10^{-9}$ M) | 4,53 | 66,4 |
| Tobramycine (10 $\mu$g/ml) + ANP ($10^{-9}$ M) | 0,7 | 94,8 |

Dans ces conditions, les inventeurs ont observé que le biofilm était fortement dispersé par la combinaison ANP (1 nM) + tobramycine (50 $\mu$g/ml), qui agit de manière synergique pour obtenir une destruction du biofilm à 96,3%. De même, la combinaison ANP (1 nM) + tobramycine (10 $\mu$g/ml), agit de manière synergique pour obtenir une destruction du biofilm à 94,8%.

**Exemple 7** : **Effet d'une combinaison d'ANP et de ciprofloxacine sur les biofilms préformés de *P. aeruginosa***

Cet exemple montre l'action synergique la combinaison ANP + ciprofloxacine sur les biofilms préformés de *P. aeruginosa.*

Matériel et méthodes

Pour étudier l'impact de l'ANP en combinaison avec la ciprofloxacine sur des biofilms préformés de 24h (comme décrit dans l'exemple 1), 300 $\mu$l d'un mélange d'ANP (à 10 nM) et de ciprofloxacine (0,01 $\mu$g/ml ou 0,04 $\mu$g/ml) ou 300 $\mu$l de ciprofloxacine seul (condition contrôle) ont été injectés dans différents canaux de la chambre à flux. Le traitement du biofilm préformé a été réalisé pendant 2 h à 37°C en conditions statiques. Après les 2 h, le flux de milieu a été de nouveau appliqué pendant 15 min afin d'éliminer les cellules qui se seraient détachées du biofilm sous l'action du traitement.
Comme décrit dans l'exemple 1, les biofilms ont été observés en microscopie confocale à balayage laser, après avoir été marqués pendant 15 min avec 5 $\mu$M de fluorochrome Syto9 green (Invitrogen).

Résultats

Les inventeurs ont étudié le potentiel d'activité anti-biofilm de la combinaison ANP (à $10^{-8}$ M ; 30 ng/ml) + ciprofloxacine (à 0,04 $\mu$g/ml ou 0,01 $\mu$g/ml) sur un biofilm établi de *P. aeruginosa.*
Le biovolume du biofilm a été déterminé et les résultats sont décrits dans les tableaux ci-dessous.

| Conditions expérimentales | Biovolume ($\mu m^3/\mu m^2$) | % inhibition |
|---|---|---|
| PA14 non traitées | 12,6 | |
| Ciprofloxacine (0,04 $\mu$g/ml) | 0,7 | 99,3 |
| Ciprofloxacine (0,04 $\mu$g/ml) + ANP ($10^{-8}$ M) | 0,5 | 99,5 |

| Conditions expérimentales | Biovolume ($\mu m^3/\mu m^2$) | % inhibition |
|---|---|---|
| PA14 non traitées | 15,5 | |
| Ciprofloxacine (0,01 $\mu$g/ml) | 1,55 | 90,0 |
| Ciprofloxacine (0,01 $\mu$g/ml) + ANP ($10^{-8}$ M) | 0,47 | 97,0 |

Dans ces conditions, les inventeurs ont observé que le biofilm était encore plus fortement dispersé par la combinaison ANP + ciprofloxacine, comparé au traitement avec l'antibiotique ciprofloxacine seul.
Ainsi, la combinaison avec l'ANP permet d'obtenir une destruction de 97% du biofilm en combinaison avec une concentration de 0,01 $\mu$g/ml de ciprofloxacine.

**Exemple 8 : Effet d'une combinaison d'ANP et de la colistine sur les biofilms préformés de *P. aeruginosa***

Cet exemple montre l'action synergique la combinaison ANP + colistine sur les biofilms préformés de *P. aeruginosa.*

Matériel et méthodes

Pour étudier l'impact de l'ANP en combinaison avec la colistine sur des biofilms préformés de 24h (comme décrit dans l'exemple 1), 300 $\mu$l d'un mélange d'ANP (à 10 nM) et de colistine (1 $\mu$g/ml) ou 300 $\mu$l de colistine seule (condition contrôle) ont été injectés dans différents canaux de la chambre à flux. Le traitement du biofilm préformé a été réalisé pendant 2 h à 37°C en conditions statiques. Après les 2 h, le flux de milieu a été de nouveau appliqué pendant 15 min afin d'éliminer les cellules qui se seraient détachées du biofilm sous l'action du traitement.

Comme décrit dans l'exemple 1, les biofilms ont été observés en microscopie confocale à balayage laser, après avoir été marqués pendant 15 min avec 5 $\mu$M de fluorochrome Syto9 green (Invitrogen).

Résultats

Les inventeurs ont étudié le potentiel d'activité anti-biofilm de la combinaison ANP (à $10^{-8}$ M ; 30 ng/ml) + colistine (à 1 $\mu$g/ml) sur un biofilm établi de *P. aeruginosa.*

Le biovolume du biofilm a été déterminé et les résultats sont décrits dans les tableaux ci-dessous.

| Conditions expérimentales | Biovolume ($\mu$m$^3$/$\mu$m$^2$) | % inhibition |
|---|---|---|
| **PA14 non traitées** | 18,5 | |
| **ANP ($10^{-8}$ M)** | 4,9 | 73,5 |
| **Colistine (1 $\mu$g/ml)** | 2,1 | 88,6 |
| **Colistine (1 $\mu$g/ml) + ANP ($10^{-8}$ M)** | 0,5 | 97,3 |

Dans ces conditions, les inventeurs ont observé que le biofilm était encore plus fortement dispersé par la combinaison ANP + colistine (1 $\mu$g/ml), comparé au traitement avec l'antibiotique colistine seul ou au traitement à l'ANP seul.

Ainsi, la combinaison avec l'ANP permet d'obtenir une destruction de 97,3 % du biofilm en combinaison avec une concentration de 1 $\mu$g/ml de colistine.

**Exemple 9 : Effet d'une combinaison d'ANP et de l'Imipenem sur les biofilms préformés de *P. aeruginosa***

Cet exemple montre l'action synergique la combinaison ANP + Imipenem sur les biofilms préformés de *P. aeruginosa.*

Matériel et méthodes

Pour étudier l'impact de l'ANP en combinaison avec l'Imipenem sur des biofilms préformés de 24h (comme décrit dans l'exemple 1), 300 $\mu$l d'un mélange d'ANP (à 10 nM) et d'Imipenem (0,5 $\mu$g/ml) ou 300 $\mu$l d'Imipenem seul (condition contrôle) ont été injectés dans différents canaux de la chambre à flux. Le traitement du biofilm préformé a été réalisé pendant 2 h à 37°C en conditions statiques. Après les 2 h, le flux de milieu a été de nouveau appliqué pendant 15 min afin d'éliminer les cellules qui se seraient détachées du biofilm sous l'action du traitement.

Comme décrit dans l'exemple 1, les biofilms ont été observés en microscopie confocale à balayage laser, après avoir été marqués pendant 15 min avec 5 $\mu$M de fluorochrome Syto9 green (Invitrogen).

Résultats

Les inventeurs ont étudié le potentiel d'activité anti-biofilm de la combinaison ANP (à $10^{-8}$ M ; 30 ng/ml) + Imipenem (à 0,5 $\mu$g/ml) sur un biofilm établi de *P. aeruginosa.*

Le biovolume du biofilm a été déterminé et les résultats sont décrits dans les tableaux ci-dessous.

| Conditions expérimentales | Biovolume ($\mu m^3/\mu m^2$) | % inhibition |
|---|---|---|
| PA14 non traitées | 28,0 | |
| ANP ($10^{-8}$ M) | 6,9 | 75,4 |
| Imipenem (0,5 $\mu$g/ml) | 7,0 | 75,0 |
| Imipenem (0.5 $\mu$g/ml) + ANP ($10^{-8}$ M) | 0,6 | 97,9 |

Dans ces conditions, les inventeurs ont observé que le biofilm était encore plus fortement dispersé par la combinaison ANP + Imipenem (0,5 $\mu$g/ml), comparé au traitement avec l'antibiotique Imipenem seul ou au traitement à l'ANP seul.

Ainsi, la combinaison avec l'ANP permet d'obtenir une destruction de 97,9 % du biofilm en combinaison avec une concentration de 0,5 $\mu$g/ml d'Imipenem.

**Exemple 10** : **Effet d'une combinaison d'ANP et de polymyxine B sur les biofilms préformés de *P. aeruginosa***

Cet exemple montre l'action synergique la combinaison ANP + Polymyxine B sur les biofilms préformés de *P. aeruginosa.*

Matériel et méthodes

Pour étudier l'impact de l'ANP en combinaison avec la polymyxine B sur des biofilms préformés de 24h (comme décrit dans l'exemple 1), 300 $\mu$l d'un mélange d'ANP (à 10 nM) et de Polymyxine B (4 $\mu$g/ml) ou 300 $\mu$l de Polymyxine B seul (condition contrôle) ont été injectés dans différents canaux de la chambre à flux. Le traitement du biofilm préformé a été réalisé pendant 2 h à 37°C en conditions statiques. Après les 2 h, le flux de milieu a été de nouveau appliqué pendant 15 min afin d'éliminer les cellules qui se seraient détachées du biofilm sous l'action du traitement.

Comme décrit dans l'exemple 1, les biofilms ont été observés en microscopie confocale à balayage laser, après avoir été marqués pendant 15 min avec 5 $\mu$M de fluorochrome Syto9 green (Invitrogen).

Résultats

Les inventeurs ont étudié le potentiel d'activité anti-biofilm de la combinaison ANP (à $10^{-8}$ M ; 30 ng/ml) + Polymyxine B (à 4 $\mu$g/ml) sur un biofilm établi de *P. aeruginosa.*

Le biovolume du biofilm a été déterminé et les résultats sont décrits dans les tableaux ci-dessous.

| Conditions expérimentales | Biovolume ($\mu m^3/\mu m^2$) | % inhibition |
|---|---|---|
| PA14 non traitées | 18,2 | |
| ANP ($10^{-8}$ M) | 4,5 | 75,3 |
| Polymyxine B (4 $\mu$g/ml) | 7,4 | 59,3 |
| Polymyxine B (4 $\mu$g/ml) + ANP ($10^{-8}$ M) | 3,0 | 83,5 |

Dans ces conditions, les inventeurs ont observé que le biofilm était encore plus fortement dispersé par la combinaison ANP + Polymyxine B (4 $\mu$g/ml), comparé au traitement avec l'antibiotique Polymyxine B seul ou au traitement à l'ANP seul.

Ainsi, la combinaison avec l'ANP permet d'obtenir une destruction de 83,5 % du biofilm en combinaison avec une concentration de 4 $\mu$g/ml de Polymyxine B.

**Exemple 11** : **Effet d'un traitement séquentiel d'ANP puis de tobramycine sur les biofilms préformés de *P. aeruginosa.***

Cet exemple montre l'action séquentielle du traitement par l'ANP suivi d'un traitement à la tobramycine sur les biofilms préformés de *P. aeruginosa.*

Matériel et méthodes

Le matériel et méthodes utilisé dans cet exemple est identique à celui décrit ci-dessus dans l'exemple 1, avec les précisions suivantes :

Pour étudier l'impact d'une exposition séquentielle à l'ANP puis à la tobramycine sur des biofilms préformés de 24 h (comme décrit dans l'exemple 1), 300 $\mu$l d'ANP (à 1 nM) (condition contrôle) ont été injectés dans deux canaux de la chambre à flux. Le traitement du biofilm préformé a été réalisé pendant 2 h à 37°C en conditions statiques. Après les 2 h, le flux de milieu a été de nouveau appliqué pendant 15 min afin d'éliminer les cellules qui se seraient détachées du biofilm sous l'action du traitement. Puis 300 $\mu$l de tobramycine (50 $\mu$g/ml) ou 300 $\mu$l d'eau milliQ stérile (condition contrôle) ont été injectés des deux canaux de la chambre à flux, pré-exposé à l'ANP. Le second traitement du biofilm préformé a été réalisé à nouveau pendant 2 h à 37°C en conditions statiques. Après les 2 h, le flux de milieu a été de nouveau appliqué pendant 15 min afin d'éliminer les cellules qui se seraient détachées du biofilm sous l'action du traitement.

Comme décrit dans l'exemple 1, les biofilms ont été observés en microscopie confocale à balayage laser, après avoir été marqués pendant 15 min avec 5 $\mu$M de fluorochrome Syto9 green (Invitrogen).

Résultats

Les inventeurs ont étudié le potentiel d'activité anti-biofilm de l'exposition séquentielle d'ANP (à $10^{-9}$ M ; 3 ng/ml) puis de tobramycine (à 50 $\mu$g/ml) sur un biofilm établi de *P. aeruginosa.*

Le biovolume du biofilm a été déterminé et les résultats sont décrits dans les tableaux ci-dessous.

| Conditions expérimentales | Biovolume ($\mu$m$^3$/$\mu$m$^2$) | % inhibition |
|---|---|---|
| **PA14 non traitées** | 13,1 | |
| **ANP ($10^{-9}$ M)** | 4,1 | 68,7 |
| **ANP ($10^{-9}$ M) (2h) puis Tobramycine (50 $\mu$g/ml) (2h)** | 0,7 | 94,7 |

Dans ces conditions, les inventeurs ont observé que le biofilm était encore plus fortement dispersé par un double traitement séquentiel ANP ($10^{-9}$ M) (2h) puis tobramycine (50 $\mu$g/ml) (2h) comparé au traitement avec l'ANP seul.

Ainsi, le traitement séquentiel ANP (2h) puis tobramycine (2h) permet d'obtenir une destruction de 94,7 % du biofilm.

**Revendications**

**1.** Peptide natriurétique choisi dans le groupe constitué de (i) l'ostéocrine, un propeptide d'ostéocrine ou dérivé d'ostéocrine, (ii) une lébétine, un fragment de lébétine ou un dérivé de lébétine ou d'un fragment de lébétine, et (iii) un peptide ANP, propeptide d'ANP ou dérivé de peptide ANP, pour son utilisation dans une méthode de traitement thérapeutique d'une infection bactérienne associée à un biofilm bactérien chez un sujet,

dans laquelle ledit peptide natriurétique disperse ledit biofilm bactérien,
dans laquelle le dérivé d'ostéocrine est une ostéocrine dans laquelle un ou plusieurs résidus d'acide aminé a été modifié chimiquement ou un variant peptidique d'ostéocrine,
dans laquelle le dérivé de lébétine ou d'un fragment de lébétine est une lébétine ou un fragment de lébétine dans laquelle/lequel un ou plusieurs résidus d'acide aminé a été modifié chimiquement ou un variant peptidique de lébétine ou de fragment de lébétine, et dans laquelle ledit dérivé de peptide ANP est une forme tronquée d'ANP correspondant aux acides aminés 4 ou 5 à 28 de la forme entière de l'ANP, une forme ayant une structure qui est un dimère antiparallèle de l'ANP, un polymère constitué de monomères d'ANP, un peptide ANP dans lequel 1 à 4 acides aminés ont été supprimés, substitués ou ajoutés, un peptide ANP dans lequel a été substitué l'acide aminé Phe de la boucle en position 8, un peptide ANP dans lequel a été substitué l'acide aminé Phe en position 26 ou l'acide aminé Ser en position 25, l'urodilatine, le fsANP ou une version linéarisée de l'ANP avec clivage du pont disulfure.

**2.** Peptide natriurétique pour son utilisation selon la revendication 1, dans laquelle ladite infection bactérienne est une infection *à Pseudomonas aeruginosa.*

**3.** Peptide natriurétique pour son utilisation selon la revendication 1 ou 2, dans laquelle ledit sujet souffre d'une pathologie respiratoire chronique, en particulier de la mucoviscidose.

4.  Peptide natriurétique pour son utilisation selon la revendication 1 ou 2, dans laquelle ledit sujet porte un implant ou une prothèse, ou souffre de brûlures importantes (grand brûlé) ou d'une infection de surface.

5.  Peptide natriurétique pour son utilisation selon l'une quelconque des revendications 1 à 4, dans laquelle ledit peptide natriurétique est utilisé en combinaison avec un antibiotique.

6.  Peptide natriurétique pour son utilisation selon la revendication 5, dans laquelle ledit antibiotique est un antibiotique aminoside ou une quinolone.

7.  Peptide natriurétique pour son utilisation selon l'une des revendications 1 à 6, dans laquelle ledit peptide natriurétique est utilisé en combinaison avec au moins un autre peptide natriurétique.

8.  Composition pharmaceutique comprenant (A) un peptide natriurétique choisi dans le groupe constitué d'une lébétine, un fragment de lébétine et un dérivé de lébétine ou d'un fragment de lébétine, et (B) un antibiotique, dans laquelle le dérivé de lébétine ou d'un fragment de lébétine est une lébétine ou un fragment de lébétine dans laquelle/lequel un ou plusieurs résidus d'acide aminé a été modifié chimiquement ou un variant peptidique.

9.  Combinaison pharmaceutique antibactérienne comprenant (A) un peptide natriurétique choisi dans le groupe constitué de (i) l'ostéocrine, un propeptide d'ostéocrine ou dérivé d'ostéocrine, (ii) une lébétine, un fragment de lébétine ou un dérivé de lébétine ou d'un fragment de lébétine, et (iii) un peptide ANP, propeptide d'ANP ou dérivé de peptide ANP, et (B) un antibiotique pour une utilisation simultanée, séparée ou séquentielle dans le traitement thérapeutique d'une infection bactérienne associée à un biofilm bactérien chez un sujet,

    dans laquelle ledit peptide natriurétique disperse ledit biofilm bactérien,
    dans laquelle le dérivé d'ostéocrine est une ostéocrine dans laquelle un ou plusieurs résidus d'acide aminé a été modifié chimiquement ou un variant peptidique d'ostéocrine,
    dans laquelle le dérivé de lébétine ou d'un fragment de lébétine est une lébétine ou un fragment de lébétine dans laquelle/lequel un ou plusieurs résidus d'acide aminé a été modifié chimiquement ou un variant peptidique de lébétine ou de fragment de lébétine, et
    dans laquelle ledit dérivé de peptide ANP est une forme tronquée d'ANP correspondant aux acides aminés 4 ou 5 à 28 de la forme entière de l'ANP, une forme ayant une structure qui est un dimère antiparallèle de l'ANP, un polymère constitué de monomères d'ANP, un peptide ANP dans lequel 1 à 4 acides aminés ont été supprimés, substitués ou ajoutés, un peptide ANP dans lequel a été substitué l'acide aminé Phe de la boucle en position 8, un peptide ANP dans lequel a été substitué l'acide aminé Phe en position 26 ou l'acide aminé Ser en position 25, l'urodilatine, le fsANP ou une version linéarisée de l'ANP avec clivage du pont disulfure.

10. Utilisation *in vitro* ou *ex vivo* d'un peptide natriurétique choisi dans le groupe constitué de (i) l'ostéocrine, un propeptide d'ostéocrine ou dérivé d'ostéocrine, (ii) une lébétine, un fragment de lébétine ou un dérivé de lébétine ou d'un fragment de lébétine, et (iii) un peptide ANP, d'un propeptide d'ANP ou d'un dérivé de peptide ANP, pour disperser un biofilm bactérien,

    dans laquelle le dérivé d'ostéocrine est une ostéocrine dans laquelle un ou plusieurs résidus d'acide aminé a été modifié chimiquement ou un variant peptidique d'ostéocrine,
    dans laquelle le dérivé de lébétine ou d'un fragment de lébétine est une lébétine ou un fragment de lébétine dans laquelle/lequel un ou plusieurs résidus d'acide aminé a été modifié chimiquement ou un variant peptidique de lébétine ou de fragment de lébétine, et
    dans laquelle ledit dérivé de peptide ANP est une forme tronquée d'ANP correspondant aux acides aminés 4 ou 5 à 28 de la forme entière de l'ANP, une forme ayant une structure qui est un dimère antiparallèle de l'ANP, un polymère constitué de monomères d'ANP, un peptide ANP dans lequel 1 à 4 acides aminés ont été supprimés, substitués ou ajoutés, un peptide ANP dans lequel a été substitué l'acide aminé Phe de la boucle en position 8, un peptide ANP dans lequel a été substitué l'acide aminé Phe en position 26 ou l'acide aminé Ser en position 25, l'urodilatine, le fsANP ou une version linéarisée de l'ANP avec clivage du pont disulfure.

**Patentansprüche**

1. Natriuretisches Peptid, ausgewählt aus der Gruppe, bestehend aus (i) Osteokrin, einem Propeptid von Osteokrin oder einem Derivat von Osteokrin, (ii) einem Lebetin, einem Lebetin-Fragment oder einem Derivat von Lebetin oder einem Lebetin-Fragment und (iii) einem ANP-Peptid, Propeptid von ANP oder ANP-Peptid-Derivat, zur Verwendung in einem Verfahren zur therapeutischen Behandlung einer mit einem bakteriellen Biofilm assoziierten bakteriellen Infektion bei einem Individuum,

   wobei das natriuretische Peptid den bakteriellen Biofilm dispergiert,
   wobei das Derivat von Osteokrin ein Osteokrin, in dem ein oder mehrere Aminosäurereste chemisch modifiziert wurden, oder eine Peptidvariante von Osteokrin ist,
   wobei das Derivat von Lebetin oder einem Lebetin-Fragment ein Lebetin oder ein Lebetin-Fragment, in dem ein oder mehrere Aminosäurereste chemisch modifiziert wurden, oder eine Peptidvariante von Lebetin oder dem Lebetin-Fragment ist, und
   wobei das ANP-Peptid-Derivat eine verkürzte Form von ANP, die den Aminosäuren 4 oder 5 bis 28 der Vollängenform des ANP entspricht, eine Form mit einer Struktur, die ein antiparalleles Dimer von ANP ist, ein Polymer, das aus ANP-Monomeren aufgebaut ist, ein ANP-Peptid, in dem 1 bis 4 Aminosäuren deletiert, substituiert oder hinzugefügt wurden, ein ANP-Peptid, in dem die Aminosäure Phe der Schleife an Position 8 substituiert wurde, ein ANP-Peptid, in dem die Aminosäure Phe an Position 26 oder die Aminosäure Ser an Position 25 substituiert wurde, Urodilatin, fsANP oder eine linearisierte Version von ANP mit Spaltung der Disulfidbrücke ist.

2. Natriuretisches Peptid zur Verwendung nach Anspruch 1, wobei die bakterielle Infektion eine Infektion mit *Pseudomonas aeruginosa* ist.

3. Natriuretisches Peptid zur Verwendung nach Anspruch 1 oder 2, wobei das Individuum an einer chronischen Atemwegserkrankung, insbesondere Mukoviszidose, leidet.

4. Natriuretisches Peptid zur Verwendung nach Anspruch 1 oder 2, wobei das Individuum ein Implantat oder eine Prothese trägt oder an erheblichen Verbrennungen (schweren Verbrennungen) oder an einer Oberflächeninfektion leidet.

5. Natriuretisches Peptid für die Verwendung nach einem der Ansprüche 1 bis 4, wobei das natriuretische Peptid in Kombination mit einem Antibiotikum verwendet wird.

6. Natriuretisches Peptid zur Verwendung nach Anspruch 5, wobei das Antibiotikum ein Aminosid-Antibiotikum oder ein Chinolon ist.

7. Natriuretisches Peptid zur Verwendung nach einem der Ansprüche 1 bis 6, wobei das natriuretische Peptid in Kombination mit mindestens einem anderen natriuretischen Peptid verwendet wird.

8. Pharmazeutische Zusammensetzung, umfassend (A) ein natriuretisches Peptid, ausgewählt aus der Gruppe bestehend aus einem Lebetin, einem Lebetin-Fragment und einem Derivat von Lebetin oder einem Lebetin-Fragment, und (B) einem Antibiotikum,
   wobei das Derivat von Lebetin oder einem Lebetin-Fragment ein Lebetin oder ein Lebetin-Fragment, in dem ein oder mehrere Aminosäurereste chemisch modifiziert wurden, oder eine Peptidvariante ist.

9. Antibakterielle pharmazeutische Kombination, umfassend (A) ein natriuretisches Peptid, ausgewählt aus der Gruppe, bestehend aus (i) Osteokrin, einem Propeptid von Osteokrin oder einem Derivat von Osteokrin, (ii) einem Lebetin, einem Lebetin-Fragment oder einem Derivat von Lebetin oder einem Lebetin-Fragment und (iii) einem ANP-Peptid, Propeptid von ANP oder ANP-Peptid-Derivat, und (B) ein Antibiotikum, zur gleichzeitigen, separaten oder sequenziellen Verwendung bei der therapeutischen Behandlung einer mit einem bakteriellen Biofilm assoziierten bakteriellen Infektion bei einem Individuum,

   wobei das natriuretische Peptid den bakteriellen Biofilm dispergiert,
   wobei das Derivat von Osteokrin ein Osteokrin, in dem ein oder mehrere Aminosäurereste chemisch modifiziert wurden, oder eine Peptidvariante von Osteokrin ist,
   wobei das Derivat von Lebetin oder einem Lebetin-Fragment ein Lebetin oder ein Lebetin-Fragment, in dem ein oder mehrere Aminosäurereste chemisch modifiziert wurden, oder eine Peptidvariante von Lebetin oder dem Lebetin-Fragment ist, und

wobei das ANP-Peptid-Derivat eine verkürzte Form von ANP, die den Aminosäuren 4 oder 5 bis 28 der Vollängenform des ANP entspricht, eine Form mit einer Struktur, die ein antiparalleles Dimer von ANP ist, ein Polymer, das aus ANP-Monomeren aufgebaut ist, ein ANP-Peptid, in dem 1 bis 4 Aminosäuren deletiert, substituiert oder hinzugefügt wurden, ein ANP-Peptid, in dem die Aminosäure Phe der Schleife an Position 8 substituiert wurde, ein ANP-Peptid, in dem die Aminosäure Phe an Position 26 oder die Aminosäure Ser an Position 25 substituiert wurde, Urodilatin, fsANP oder eine linearisierte Version von ANP mit Spaltung der Disulfidbrücke ist.

10. *In-vitro-* oder Ex-vivo-Verwendung eines natriuretischen Peptids, ausgewählt aus der Gruppe, bestehend aus (i) Osteokrin, einem Propeptid von Osteokrin oder einem Derivat von Osteokrin, (ii) einem Lebetin, einem Lebetin-Fragment oder einem Derivat von Lebetin oder einem Lebetin-Fragment und (iii) einem ANP-Peptid, einem Propeptid von ANP oder einem ANP-Peptid-Derivat, zum Dispergieren eines bakteriellen Biofilms,

wobei das Derivat von Osteokrin ein Osteokrin, in dem ein oder mehrere Aminosäurereste chemisch modifiziert wurden, oder eine Peptidvariante von Osteokrin ist,

wobei das Derivat von Lebetin oder einem Lebetin-Fragment ein Lebetin oder ein Lebetin-Fragment, in dem ein oder mehrere Aminosäurereste chemisch modifiziert wurden, oder eine Peptidvariante von Lebetin oder dem Lebetin-Fragment ist, und

wobei das ANP-Peptid-Derivat eine verkürzte Form von ANP, die den Aminosäuren 4 oder 5 bis 28 der Vollängenform des ANP entspricht, eine Form mit einer Struktur, die ein antiparalleles Dimer von ANP ist, ein Polymer, das aus ANP-Monomeren aufgebaut ist, ein ANP-Peptid, in dem 1 bis 4 Aminosäuren deletiert, substituiert oder hinzugefügt wurden, ein ANP-Peptid, in dem die Aminosäure Phe der Schleife an Position 8 substituiert wurde, ein ANP-Peptid, in dem die Aminosäure Phe an Position 26 oder die Aminosäure Ser an Position 25 substituiert wurde, Urodilatin, fsANP oder eine linearisierte Version von ANP mit Spaltung der Disulfidbrücke ist.

**Claims**

1. Natriuretic peptide selected from the group consisting of (i) osteocrin, an osteocrin propeptide or osteocrin derivative, (ii) a lebetin, a lebetin fragment or a derivative of lebetin or of a lebetin fragment, and (iii) an ANP peptide, ANP propeptide or ANP peptide derivative, for use thereof in a method for the therapeutic treatment of a bacterial infection associated with a bacterial biofilm in a subject,

wherein said natriuretic peptide disperses said bacterial biofilm,
wherein the osteocrin derivative is an osteocrin in which one or more amino acid residues have been chemically modified or an osteocrin peptide variant,
wherein the derivative of lebetin or of a lebetin fragment is a lebetin or a lebetin fragment in which one or more amino acid residues have been chemically modified or a lebetin or lebetin fragment peptide variant, and
wherein said ANP peptide derivative is a truncated form of ANP corresponding to amino acids 4 or 5 to 28 of the full form of ANP, a form having a structure which is an antiparallel dimer of ANP, a polymer consisting of monomers of ANP, an ANP peptide in which 1 to 4 amino acids have been deleted, substituted or added, an ANP peptide in which the amino acid Phe of the loop at position 8 has been substituted, an ANP peptide in which the amino acid Phe at position 26 or the amino acid Ser at position 25 has been substituted, urodilatin, fsANP or a linearized version of ANP with cleavage of the disulfide bridge.

2. Natriuretic peptide for use thereof according to Claim 1, wherein said bacterial infection is a *Pseudomonas aeruginosa* infection.

3. Natriuretic peptide for use thereof according to Claim 1 or 2, wherein said subject is suffering from a chronic respiratory disease, in particular cystic fibrosis.

4. Natriuretic peptide for use thereof according to Claim 1 or 2, wherein said subject carries an implant or wears a prosthesis, or is suffering from significant burns (serious burn victim) or from a surface infection.

5. Natriuretic peptide for use thereof according to any one of Claims 1 to 4, wherein said natriuretic peptide is used in combination with an antibiotic.

6. Natriuretic peptide for use thereof according to Claim 5, wherein said antibiotic is an aminoglycoside antibiotic or a quinolone.

7. Natriuretic peptide for use thereof according to one of Claims 1 to 6, wherein said natriuretic peptide is used in combination with at least one other natriuretic peptide.

8. Pharmaceutical composition comprising (A) a natriuretic peptide selected from the group consisting of a lebetin, a lebetin fragment and a derivative of lebetin or of a lebetin fragment, and (B) an antibiotic, wherein the derivative of lebetin or of a lebetin fragment is a lebetin or a lebetin fragment in which one or more amino acid residues have been chemically modified or a peptide variant.

9. Antibacterial pharmaceutical combination comprising (A) a natriuretic peptide selected from the group consisting of (i) osteocrin, an osteocrin propeptide or osteocrin derivative, (ii) a lebetin, a lebetin fragment or a derivative of lebetin or of a lebetin fragment, and (iii) an ANP peptide, ANP propeptide or ANP peptide derivative, and (B) an antibiotic for simultaneous, separate or sequential use in the therapeutic treatment of a bacterial infection associated with a bacterial biofilm in a subject,

  wherein said natriuretic peptide disperses said bacterial biofilm,
  wherein the osteocrin derivative is an osteocrin in which one or more amino acid residues have been chemically modified or an osteocrin peptide variant,
  wherein the derivative of lebetin or of a lebetin fragment is a lebetin or a lebetin fragment in which one or more amino acid residues have been chemically modified or a lebetin or lebetin fragment peptide variant, and
  wherein said ANP peptide derivative is a truncated form of ANP corresponding to amino acids 4 or 5 to 28 of the full form of ANP, a form having a structure which is an antiparallel dimer of ANP, a polymer consisting of monomers of ANP, an ANP peptide in which 1 to 4 amino acids have been deleted, substituted or added, an ANP peptide in which the amino acid Phe of the loop at position 8 has been substituted, an ANP peptide in which the amino acid Phe at position 26 or the amino acid Ser at position 25 has been substituted, urodilatin, fsANP or a linearized version of ANP with cleavage of the disulfide bridge.

10. *In vitro* or ex *vivo* use of a natriuretic peptide selected from the group consisting of (i) osteocrin, an osteocrin propeptide or osteocrin derivative, (ii) a lebetin, a lebetin fragment or a derivative of lebetin or of a lebetin fragment, and (iii) an ANP peptide, an ANP propeptide or an ANP peptide derivative, for dispersing a bacterial biofilm,

  wherein the osteocrin derivative is an osteocrin in which one or more amino acid residues have been chemically modified or an osteocrin peptide variant,
  wherein the derivative of lebetin or of a lebetin fragment is a lebetin or a lebetin fragment in which one or more amino acid residues have been chemically modified or a lebetin or lebetin fragment peptide variant, and
  wherein said ANP peptide derivative is a truncated form of ANP corresponding to amino acids 4 or 5 to 28 of the full form of ANP, a form having a structure which is an antiparallel dimer of ANP, a polymer consisting of monomers of ANP, an ANP peptide in which 1 to 4 amino acids have been deleted, substituted or added, an ANP peptide in which the amino acid Phe of the loop at position 8 has been substituted, an ANP peptide in which the amino acid Phe at position 26 or the amino acid Ser at position 25 has been substituted, urodilatin, fsANP or a linearized version of ANP with cleavage of the disulfide bridge.

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Littérature non-brevet citée dans la description**

- **SANTAJIT** ; **INDRAWATTANA**. *Biomed. Res. Int.*, 2016, vol. 2016, 2475067
- **LESOUHAITIER et al.** *J. Innate Immun*, 2019, vol. 11, 227-241
- The natriuretic peptide hormones prevent Pseudomonas aeruginosa biofilm formation through a specific bacterial target.. **DESRIAC et al.** 16th international conference on Pseudomonas, Sep 2017, Liverpool, United Kingdom. September 2017
- **ROSAY et al.** *MBio*, 2015, vol. 6, 6
- **DESRIAC et al.** *Pathogens*, 2018, vol. 7, 47
- **ROSAY et al.** *mBio*, 2015, vol. 25, e01033-15
- **KAWAKOSHI et al.** *J. Mol. Endocrinol.*, 2004, vol. 32, 547-555
- **SCHWEITZ et al.** *J. Biol. Chem.*, 1992, vol. 267, 13928-13932
- **SRIDHARAN et al.** *Biochem. J*, 2015, vol. 469, 255-266
- **FRY et al.** *Proc. Natl. Acad. Sci. USA*, 2009, vol. 106, 8969-8974
- **ALVES et al.** *Toxicon*, 2013, vol. 74, 19-26
- **TAKEI et al.** *General and Comparative Endocrinology*, 2011, vol. 171, 258-266
- **ICHIKI** ; **BURNETT**. *Circ J.*, 2017, vol. 81, 913-919
- **DICKEY et al.** *J Biol Chem*, 2009, vol. 284, 19196-19202
- **BARBOUCHE et al.** *FEBS Lett*, 1996, vol. 392, 6-10
- **BARBOUCHE et al.** *Toxicon*, 1998, vol. 36 (12), 1939-47
- **LIBERATI et al.** *Proc. Natl. Acad. Sci. USA*, 2006, vol. 103, 2833-2838
- **BAZIRE et al.** *J. Bacteriol.*, 2010, vol. 192, 3001-3010
- **HEYDORN et al.** *Microbiology*, 2000, vol. 146, 2395-2407